# EUROPEAN PATENT APPLICATION

(11) **EP 4 725 482 A1**
(43) Date of publication of application: **15.04.2026**
(21) Application number: 24818691.8
(22) Date of filing: 05.06.2024
(51) Int. Cl.: A61K 31/4155, A61P 25/28

(54) **GLUCOKINASE ACTIVATOR FOR COGNITIVE DISORDERS AND NEURODEGENERATIVE DISEASES**

(30) Priority: 06.06.2023 CN 202310662626
(71) Applicant: Hua Medicine (Shanghai) Ltd., Shanghai 201203 (CN)
(72) Inventor: CHEN, Li, Shanghai 201203 (CN); NI, Jiangxia, Shanghai 201203 (CN); FENG, Lingge, Shanghai 201203 (CN)
(74) Representative: Cabinet Becker et Associés
(86) International application number: PCT/CN2024/097577
(87) International publication number: WO 2024/251159

(57) **Abstract**

A use of a glucokinase activator or a prodrug thereof, or a pharmaceutically acceptable salt, an isotopic label, a crystal form, a hydrate, a solvate, a diastereomer or an enantiomer in preventing, alleviating or treating cognitive disorders or neurodegenerative diseases in subjects, or delaying the progress of cognitive disorders or neurodegenerative diseases in subjects, or reducing the risk of cognitive disorders or neurodegenerative diseases in subjects.

## Description

The present disclosure claims the priority of Chinese invention patent application CN202310662626.8, filed on June 6, 2023, which is incorporated herein by reference in its entirety as part of this disclosure.

### TECHNICAL FIELD

The present disclosure relates to a glucokinase activator for cognitive impairment and neurodegenerative diseases.

### BACKGROUND

Type 2 diabetes mellitus (T2DM) is the most prevalent metabolic disease, and its incidence is increasing year by year, making it a public health problem that urgently needs to be addressed worldwide. Regarding the secondary complications of T2DM, increasing research evidence links T2DM with cognitive impairment (CI) and neurodegenerative diseases, but the underlying pathology behind these secondary complications is not yet fully understood. Many studies have shown that diabetic patients, especially those with T2DM, have a higher risk of developing Alzheimer's disease (AD) (AM Nazareth, Dementia & neuropsychologia (2017); Jun; 11(2): 105-113; G. Cooray et al., Psychoneuroendocrinology. sychoneuroendocrinology (2011); 36, 77-86). Some studies have even referred to Alzheimer's disease as Type 3 diabetes mellitus (T3DM) (Suzanne M. de la Monte et al., J Diabetes Sci Technol (2008);2(6):1101-1113; Huang J et al., Frontiers in Aging Neuroscience (2023); 15:1130253).

To date, extensive work has failed to identify any pharmacological treatment that can prevent, delay, or alter the course of cognitive impairment (CI) and neurodegenerative diseases. Therefore, improved pharmacological therapies are needed for the subjects with cognitive impairment and neurodegenerative diseases.

### SUMMARY

The inventors have surprisingly discovered that administration of a glucokinase activator (e.g., Dorzagliatin) can prevent, alleviate, or treat cognitive impairment or a neurodegenerative disease, delay the progression of cognitive impairment or a neurodegenerative disease, and reduce the risk of developing cognitive impairment or a neurodegenerative disease. Furthermore, the inventors have surprisingly discovered that Dorzagliatin delayed the progression of cognitive impairment or a neurodegenerative disease (e.g., mild cognitive impairment (MCI), Alzheimer's disease) and reduced the risk of developing a cognitive impairment-related disease in an animal model.

One of the objectives of the present disclosure is to investigate the effects of a glucokinase activator on the prevention, alleviation, or treatment, as well as delaying the progression and reducing the risk of the development, etc. of cognitive impairment or a neurodegenerative disease in a subject.

In one aspect, the present disclosure provides use of a glucokinase activator or a prodrug thereof, or a pharmaceutically acceptable salt, a prodrug, an isotope-labeled form, a crystal form, a hydrate, a solvate, a diastereomer or an enantiomer thereof in the manufacture of a medicament for the prevention, alleviation or treatment of cognitive impairment or a neurodegenerative disease in a subject, or delaying the progression of cognitive impairment or a neurodegenerative disease in a subject, or reducing the risk of developing cognitive impairment or a neurodegenerative disease in a subject.

In one aspect, the present disclosure provides use of Dorzagliatin or a prodrug thereof, or a pharmaceutically acceptable salt, an isotope-labeled form, a crystal form, a hydrate, a solvate, a diastereomer or an enantiomer thereof, in the manufacture of a medicament for the prevention, alleviation or treatment of cognitive impairment or a neurodegenerative disease in a subject, or delaying the progression of cognitive impairment or a neurodegenerative disease in a subject, or reducing the risk of developing cognitive impairment or a neurodegenerative disease in a subject.

In one aspect, the present disclosure provides use of Dorzagliatin or a prodrug thereof in the manufacture of a medicament for the prevention, alleviation or treatment of cognitive impairment or a neurodegenerative disease in a subject, or delaying the progression of cognitive impairment or a neurodegenerative disease in a subject, or reducing the risk of developing cognitive impairment or a neurodegenerative disease in a subject.

In another aspect, the present disclosure provides a method of preventing, alleviating or treating cognitive impairment or a neurodegenerative disease, or delaying the progression of cognitive impairment or a neurodegenerative disease, or reducing the risk of developing cognitive impairment or a neurodegenerative disease, comprising administering a glucokinase activator or a prodrug thereof, or a pharmaceutically acceptable salt, an isotope-labeled form, a crystal form, a hydrate, a solvate, a diastereomer or an enantiomer thereof to a subject in need.

In another aspect, the present disclosure provides a method of preventing, alleviating or treating cognitive impairment or a neurodegenerative disease, or delaying the progression of cognitive impairment or a neurodegenerative disease, or reducing the risk of developing cognitive impairment or a neurodegenerative disease, comprising administering Dorzagliatin or a prodrug thereof, or a pharmaceutically acceptable salt, an isotope-labeled form, a crystal form, a hydrate, a solvate, a diastereomer or an enantiomer thereof to a subject in need.

In another aspect, the present disclosure provides a method of preventing, alleviating or treating cognitive impairment or a neurodegenerative disease, or delaying the progression of cognitive impairment or a neurodegenerative disease, or reducing the risk of developing cognitive impairment or a neurodegenerative disease, comprising administering Dorzagliatin or a prodrug thereof to a subject in need.

In another aspect, the present disclosure provides a glucokinase activator or a prodrug thereof, or a pharmaceutically acceptable salt, an isotope-labeled form, a crystal form, a hydrate, a solvate, a diastereomer or an enantiomer thereof, for use in preventing, alleviating or treating cognitive impairment or a neurodegenerative disease, or delaying the progression of cognitive impairment or a neurodegenerative disease, or reducing the risk of developing cognitive impairment or a neurodegenerative disease.

In another aspect, the present disclosure provides Dorzagliatin or a prodrug thereof, or a pharmaceutically acceptable salt, an isotope-labeled form, a crystal form, a hydrate, a solvate, a diastereomer or an enantiomer thereof, for use in preventing, alleviating or treating cognitive impairment or a neurodegenerative disease, or delaying the progression of cognitive impairment or a neurodegenerative disease, or reducing the risk of developing cognitive impairment or a neurodegenerative disease.

In another aspect, the present disclosure provides Dorzagliatin or a prodrug thereof, for use in preventing, alleviating or treating cognitive impairment or a neurodegenerative disease, or delaying the progression of cognitive impairment or a neurodegenerative disease, or reducing the risk of developing cognitive impairment or a neurodegenerative disease.

Another objective of the present disclosure is to investigate the effects of a glucokinase activator (e.g., Dorzagliatin, a prodrug of Dorzagliatin) on the prevention, alleviation, or treatment, as well as delaying the progression and reducing the risk of the development, etc. of cognitive impairment or a neurodegenerative disease in a subject. The subject has one or more indications selected from: type I diabetes, type II diabetes, maturity-onset diabetes of the young (MODY), gestational diabetes, impaired glucose tolerance, abnormal fasting blood glucose level, hyperglycemia, postprandial hyperglycemia, overweight, obesity, hypertension, cardiovascular disease, insulin resistance and/or metabolic syndrome.

### DETAILED DESCRIPTION

### Definitions

Unless otherwise specified, all technical and scientific terms used herein have the same meanings as commonly understood by those skilled in the art to which the present disclosure belongs, but in case of conflict, the definitions in this specification shall prevail.

As used in the specification and claims, the singular forms "a", "an" and "the (said)" include plural forms, unless clearly specified otherwise in the context.

All numerical values or expressions relating to component quantities used in the specification and claims should be understood to be modified by "about" in all cases. The term "about" when referring to an amount or a numerical range means that the amount or the numerical range referred to is an approximate value within experimental variability (or within statistical experimental error). Therefore, the amount or the numerical range can be varied between, for example, ±5% of the amount or the numerical range referred to.

As used in this specification and in the claims, "and/or" should be understood to mean "either or both" of the associated elements, i.e., the elements exist jointly in some cases and separately in other cases. Multiple elements listed with "and/or" should be construed in the same fashion, i.e., "one or more" of the associated elements. In addition to the elements specifically identified by the "and/or" clause, other elements may optionally exist, whether related or unrelated to those elements specifically identified. Thus, as a nonlimiting example, a reference to "A and/or B", when used in conjunction with open-ended language such as "comprising", can refer, in one embodiment, to A only (optionally including elements other than B); in another embodiment, to B only (optionally including elements other than A); in yet another embodiment, to both A and B (optionally including other elements); etc.

The abbreviations used in the present disclosure have their usual meanings in the fields of chemistry, biology, and formulation.

By "pharmaceutically available" or "pharmaceutically acceptable", it is meant a material which is not biologically or otherwise undesirable, i.e., the material may be administered to an individual without causing any undesirable biological effects or interacting in a deleterious manner with any other component of a composition comprising the material.

The term "pharmaceutically acceptable salt" refers to a salt that does not irritate an organism significantly and retains the biological activity and properties of a compound.

The term "pharmaceutically acceptable carrier" refers to an inactive ingredient that does not irritate an organism significantly and does not abrogate the biological activity and properties of an administered compound. As used herein, "carrier" and "excipient" have the same meaning.

The term "therapeutically effective amount" refers to an amount of an agent sufficient to provide a desired biological result. The result may be reduction and/or alleviation of a sign, symptom, or cause of a disease, or any other desired change of a biological system. For example, a "therapeutically effective amount" for therapeutic use refers to a necessary amount of a composition comprising a compound disclosed herein as an active ingredient for providing a clinically significant decrease in a disease. In any individual case, an appropriate "therapeutically effective amount" may be determined by one of ordinary skill in the art using routine experimentation. Thus, the expression "therapeutically effective amount" generally refers to an amount of an active substance at which it has a therapeutic effect.

The term "cognitive impairment" refers to an impairment in which animals show a decline in cognitive function or cognitive domains, such as working memory, attention and arousal, language learning and memory, visual learning and memory, reasoning and problem-solving (e.g., executive function), task processing speed and/or social cognition. Particularly, it is known that cognitive impairment may manifest as attention deficit, disorganized thinking, slowed thinking, difficulty in comprehension, poor attention, loss of problem-solving ability, disorganized memory, difficulty in expressing thoughts and/or integrating thoughts, feelings and behaviors, or difficulty in eliminating inappropriate thoughts, and may be used interchangeably with the terms "cognitive deficit", "cognitive damage", etc. Symptoms of cognitive impairment include decreased attention, decreased language ability, decreased spatial and temporal abilities, decreased reasoning ability, or decreased judgment.

The terms "treat", "prevent" and "alleviate" are intended to mean delaying disease progression, preventing disease progression and/or reducing the severity of symptoms that will develop or are expected to develop. Specifically, the term "prevention" refers to reducing or eliminating the possibility of contracting a disease, the term "alleviation" refers to alleviating all or part of a disease and/or its accompanying symptoms, and the term "treatment" refers to eliminating all or part of a disease and/or its accompanying symptoms. Thus, these terms include ameliorating existing disease symptoms, preventing additional symptoms, ameliorating or preventing the underlying metabolic causes of symptoms, inhibiting a disorder or disease, e.g., preventing the development of a disorder or disease, relieving a disorder or disease, causing a regression of a disorder or disease, relieving a condition caused by a disease or disorder, or stopping symptoms of a disease or disorder.

The term "subject" encompasses mammals and non-mammals. Examples of mammals include, but are not limited to, any member of the mammalian class: humans, non-human primates such as chimpanzees, and other apes and monkey species; farm animals such as cattle, horses, sheep, goats, swine; domestic animals such as rabbits, dogs, and cats; laboratory animals including rodents, such as rats, mice and guinea pigs, and the like. Examples of non-mammals include, but are not limited to, birds, fish and the like. In one embodiment of the present disclosure, the mammal is a human. The term "subject" includes a confirmed patient, but the "subject" does not need to have any special identity to a hospital, clinic, or research facility (e.g., as a confirmed patient, study participant, etc.).

It is to be understood that the terminology employed herein is for the purpose of describing particular embodiments, but not intended to be limiting. Further, alternative methods, devices and materials are described below, although any methods, devices and materials similar or equivalent to those described herein can be used in the practice or testing of the present disclosure.

### BRIEF DESCRIPTION OF DRAWINGS

Fig. 1 is a comparison of the fasting blood glucose concentrations at weeks 6, 8, 12, 16, 20, 29, and 31 in the Wistar rat-solvent group, GK rat-solvent group, and GK rat-Dorzagliatin group.
In the figure, comparing the GK rat-solvent group with the Wistar rat-solvent group, *P<0.05, ***P<0.001, ****P<0.0001; comparing the GK rat-Dorzagliatin group with the GK rat-solvent group, #P<0.05 and ##P<0.01.
Fig. 2 shows the average time spent in the original platform quadrant in the Morris water maze spatial memory test for the Wistar rat-solvent group, GK rat-solvent group, and GK rat-Dorzagliatin group.
In the figure, comparing the GK rat-solvent group with the Wistar rat-solvent group, **P < 0.01; comparing the GK rat-Dorzagliatin group with the GK rat-solvent group, #P < 0.05.
Fig. 3 shows the average distance traveled in the original platform quadrant in the Morris water maze spatial memory test by the Wistar rat-solvent group, the GK rat-solvent group, and the GK rat-Dorzagliatin group.
In the figure, comparing the GK rat-solvent group with the Wistar rat-solvent group, **P < 0.01; comparing the GK rat-Dorzagliatin group with the GK rat-solvent group, ##P<0.01.
Fig. 4 shows the western blot bands of two insulin receptor isoforms (IR-A protein and IR-B protein) in the hippocampus of the GK rat-solvent group and the GK rat-Dorzagliatin group and the corresponding internal reference protein GAPDH.
Fig. 5 shows the statistical analysis results of the normalized data of gray values of the IR-A protein bands in Fig. 4. In the figure, comparing the GK rat-Dorzagliatin group with the GK rat-solvent group, ***P < 0.001.
Fig. 6 shows the statistical analysis results of the normalized data of gray values of the IR-B protein bands in Fig. 4. In the figure, comparing the GK rat-Dorzagliatin group with the GK rat-solvent group, ***P < 0.001.
Fig. 7 shows the western blot bands of glucose transporters 1 and 3 (GLUT1 and GLUT3) in the hippocampus of the GK rat-solvent group and the GK rat-Dorzagliatin group and the corresponding internal reference protein GAPDH.
Fig. 8 shows the statistical analysis results of the normalized data of gray values of the GLUT1 bands in Fig. 7. In the figure, comparing the GK rat-Dorzagliatin group with the GK rat-solvent group, **P < 0.01.
Fig. 9 shows the statistical analysis results of the normalized data of gray values of the GLUT2 bands in Fig. 7. In the figure, comparing the GK rat-Dorzagliatin group with the GK rat-solvent group, ***P < 0.001.
Fig. 10 shows the western blot bands of the ionotropic glutamate receptor subunits (GluN2A and GluN2B) in the hippocampus of the GK rat-solvent group and the GK rat-Dorzagliatin group and the corresponding internal reference protein GAPDH.
Fig. 11 shows the statistical analysis results of the normalized data of gray values of the GluN2A bands in Fig. 10. In the figure, comparing the GK rat-Dorzagliatin group with the GK rat-solvent group, **P < 0.01.
Fig. 12 shows the statistical analysis results of the normalized data of gray values of the GluN2B bands in Fig. 10. In the figure, comparing the GK rat-Dorzagliatin group with the GK rat-solvent group, ****P < 0.0001.

### Specific embodiments

In some embodiments, the present disclosure relates to use of a glucokinase activator or a prodrug thereof, or a pharmaceutically acceptable salt, an isotope-labeled form, a crystal form, a hydrate, a solvate, a diastereomer or an enantiomer thereof in the manufacture of a medicament for the prevention, alleviation or treatment of cognitive impairment or a neurodegenerative disease in a subject, or delaying the progression of cognitive impairment or a neurodegenerative disease in a subject, or reducing the risk of developing cognitive impairment or a neurodegenerative disease in a subject.

In some embodiments, the glucokinase activator of the present disclosure is selected from at least one of: Dorzagliatin, AZD1656, piragliatin, AZD6370, GKA50, YH-GKA, PSN-010, LY2121260, Ganoderma lucidum polysaccharide B, eupatilin, glucolipsin A, glucolipsin B, GKM-001, TTP399, SY-004, TMG-123, AM-9514, AMG-0696, AMG-1694, AMG-3969, LCZ-960, AZD-1092, AZD5658, ARRY-403, BMS-820132, GKM-002, LY2608204, MK-0941, R-1511, RO281675, ZYGK-1, OP-986CR, CM-3, DS-7309, LY-2599506, PF-04937319, PF04991532, TAK-329, PF04991539, TAK-399, or a prodrug thereof, or a pharmaceutically acceptable salt, an isotope-labeled form, a crystal form, a hydrate, a solvate, a diastereomer or an enantiomer thereof.

In some other embodiments, the present disclosure relates to use of Dorzagliatin or a prodrug thereof, or a pharmaceutically acceptable salt, an isotope-labeled form, a crystal form, a hydrate, a solvate, a diastereomer or an enantiomer thereof, in the manufacture of a medicament for the prevention, alleviation or treatment of cognitive impairment or a neurodegenerative disease in a subject, or delaying the progression of cognitive impairment or a neurodegenerative disease in a subject, or reducing the risk of developing cognitive impairment or a neurodegenerative disease in a subject.

In some other embodiments, the glucokinase activator of the present disclosure is a compound represented by the following formula, or a pharmaceutically acceptable salt, an isotope-labeled form, a crystal form, a hydrate, a solvate, a diastereomer or an enantiomer thereof:

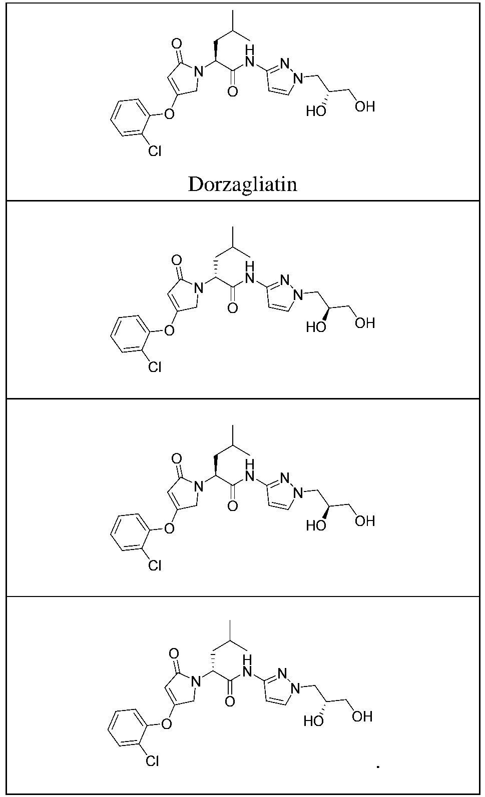

Alternatively, the glucokinase activator of the present disclosure is a compound represented by the following formula, or a pharmaceutically acceptable salt, an isotope-labeled form, a crystal form, a hydrate, or a solvate thereof:

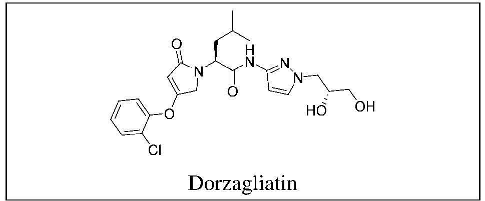

Alternatively, the glucokinase activator of the present disclosure is Dorzagliatin, or a pharmaceutically acceptable salt thereof.

In some other embodiments, the glucokinase activator of the present disclosure is a prodrug of Dorzagliatin, or a pharmaceutically acceptable salt, an isotope-labeled form, a crystal form, a hydrate, a solvate, a diastereomer or an enantiomer thereof.

Alternatively, the glucokinase activator of the present disclosure is a prodrug of Dorzagliatin, or a pharmaceutically acceptable salt thereof.

In some other embodiments, the prodrug of Dorzagliatin is a compound disclosed in WO 2023/040937 A1, or a pharmaceutically acceptable salt, an isotope-labeled form, an enantiomer or a diastereomer thereof, and the entire disclosure of the above patent application is incorporated herein by reference.

In some other embodiments, the prodrug of Dorzagliatin is a compound of formula (I), or an isotope-labeled form, an enantiomer, a diastereomer, or a pharmaceutically acceptable salt thereof: 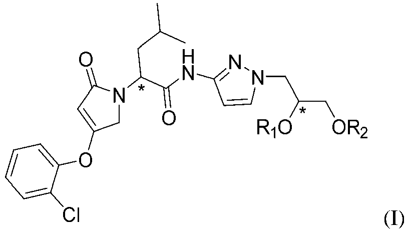 wherein
* indicates a chiral center,
R₁ is selected from H, -C(O)R₆, -C(O)OR₆, -C(O)NR₇R₈, -S(O)ₘR₆, -S(O)ₘOR₆ and -S(O)ₘNR₇R₈;
R₂ is selected from -C(O)R₃, -C(O)OR₃, -C(O)NR₄R₅, -S(O)ₘR₃, -S(O)ₘOR₃ and -S(O)ₘNR₄R₅;
or R₁ and R₂ are connected to form -CHR_{d}-, -SiR_{d}Rₑ₋, -C(O)-, -S(O)₁₋₂-, -P(O)OR_{d}-, or -CR_{d}Rₑ-CR_{d}Rₑ-;
R₃ is independently selected from H, C₁₋₆ alkyl, C₂₋₆ alkenyl, C₂₋₆ alkynyl, C₁₋₆ haloalkyl, C₃₋₇ cycloalkyl, 3- to 7-membered heterocyclyl, C₆₋₁₀ aryl and 5- to 10-membered heteroaryl, and R₃ is optionally substituted with 1, 2, 3, 4 or 5 R groups;
R₄ and R₅ are independently selected from H, C₁₋₆ alkyl, C₂₋₆ alkenyl, C₂₋₆ alkynyl and C₁₋₆ haloalkyl; or, R₄, R₅ and the N atom are taken together to form 3- to 7-membered heterocyclyl;
R₆ is independently selected from H, C₁₋₆ alkyl, C₂₋₆ alkenyl, C₂₋₆ alkynyl and C₁₋₆ haloalkyl;
R₇ and R₈ are independently selected from H, C₁₋₆ alkyl, C₂₋₆ alkenyl, C₂₋₆ alkynyl and C₁₋₆ haloalkyl; or, R₇, R₈ and the N atom are taken together to form 3- to 7-membered heterocyclyl;
R is independently selected from H, -L-halogen, -L-CN, -L-NO₂, -L-ORₐ, -L-SRₐ, -L-NR_{b}R_{c}, -L-C(O)ORₐ, -L-C(O)NR_{b}R_{c}, -L-S(O)ₘORₐ, -L-S(O)ₘNR_{b}R_{c}, C₁₋₆ alkyl, C₁₋₆ haloalkyl, C₂₋₆ alkenyl, C₂₋₆ alkynyl, C₁₋₆ haloalkyl, C₃₋₇ cycloalkyl, 3- to 7-membered heterocyclyl, C₆₋₁₀ aryl, 5- to 10-membered heteroaryl and a side chain of a natural amino acid;
wherein m = 1 or 2;
Rₐ is independently selected from H, C₁₋₆ alkyl, C₂₋₆ alkenyl, C₂₋₆ alkynyl, C₁₋₆ haloalkyl, C₃₋₇ cycloalkyl, 3- to 7-membered heterocyclyl, C₆₋₁₀ aryl and 5- to 10-membered heteroaryl;
R_{b} and R_{c} are independently selected from H, C₁₋₆ alkyl, C₂₋₆ alkenyl, C₂₋₆ alkynyl, C₁₋₆ haloalkyl, C₃₋₇ cycloalkyl, 3- to 7-membered heterocyclyl, C₆₋₁₀ aryl and 5- to 10-membered heteroaryl; or, R_{b}, R_{c} and the N atom are taken together to form 3- to 7-membered heterocyclyl;
R_{d} and Rₑ are independently selected from H, C₁₋₆ alkyl, C₂₋₆ alkenyl, C₂₋₆ alkynyl and C₁₋₆ haloalkyl; or, R_{d}, Rₑ and the C atom are taken together to form =O, =S, C₃₋₇ cycloalkyl or 3- to 7-membered heterocyclyl;
L is selected from chemical bond, -C₁₋₆ alkylene-, -C₂₋₆ alkenylene- and -C₂₋₆ alkynylene-.

In some embodiments, the prodrug of Dorzagliatin or a pharmaceutically acceptable salt thereof is selected from the compounds of the following structures:

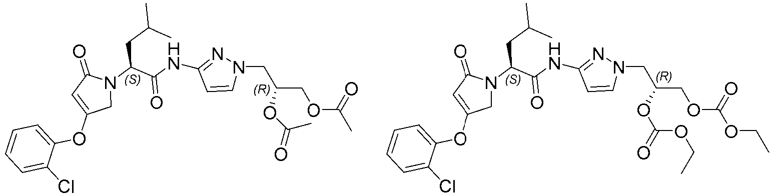

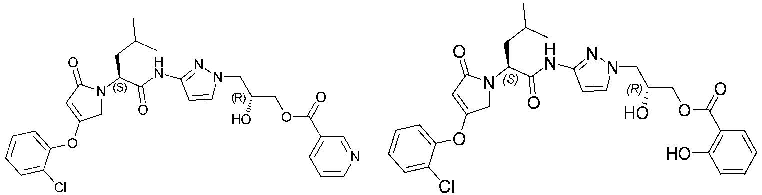

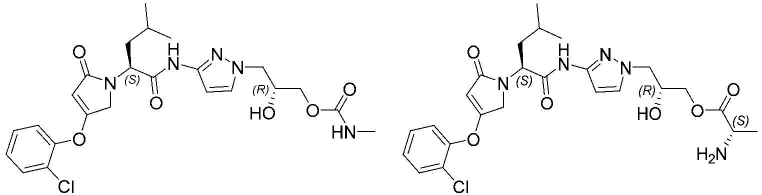

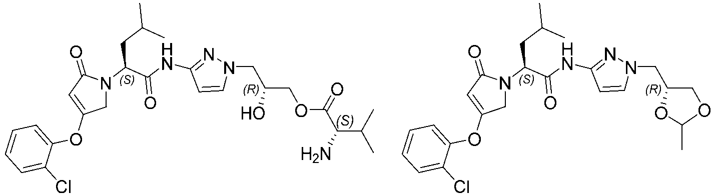

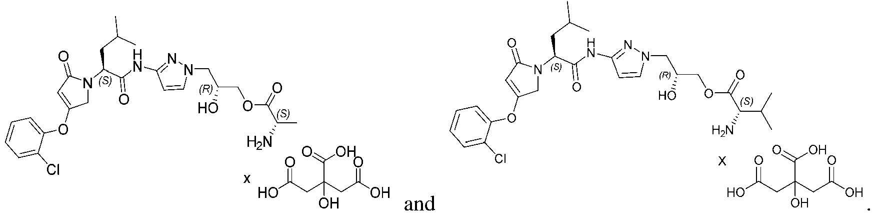

In some other embodiments, the prodrug of Dorzagliatin is a compound of the following structure:

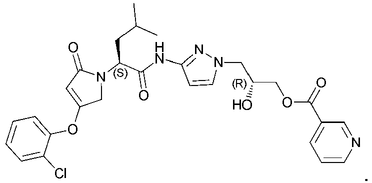

In some embodiments, symptoms of cognitive impairment include decreased attention, decreased language ability, decreased spatial and temporal abilities, decreased reasoning ability, or decreased judgment.

For example, cognitive impairment includes the following symptoms: short-term or long-term memory function, learning ability, mental flexibility, attention, executive function, spatial working memory, digital working memory, picture recognition, word recognition, checking time, mood, energy, anger, hostility, confusion, and overall mood disorders.

In some embodiments, the cognitive impairment-related disease is selected from mild cognitive impairment (MCI), preclinical Alzheimer's disease, Alzheimer's disease, dementia of Alzheimer type, presenile dementia, early-onset Alzheimer's disease, prodromal AD, senile dementia, dementia with Lewy bodies (DLB), dementia of microinfarct, AIDS-related dementia, HIV-associated dementia, Lewy body-related dementia, Down syndrome-related dementia, frontotemporal dementia, Pick's disease, recent and short-term memory impairment, age-related cognitive impairment, age-related memory impairment, drug-related cognitive impairment, immunodeficiency syndrome-related cognitive impairment, vascular disease-related cognitive impairment, schizophrenia-related cognitive impairment, Parkinson's disease-related cognitive impairment, epilepsy-related cognitive impairment, depression-related cognitive impairment, bipolar disorder-related cognitive impairment, obsessive-compulsive disorder-related cognitive impairment, post-traumatic stress disorder, attention deficit disorder, attention-deficit hyperactivity disorder, and learning disabilities.

In some embodiments, the cognitive impairment-related disease is selected from mild cognitive impairment (MCI), preclinical Alzheimer's disease, Alzheimer's disease, dementia of Alzheimer type, presenile dementia, early-onset Alzheimer's disease, prodromal AD, senile dementia, dementia with Lewy bodies, dementia of microinfarct, AIDS-related dementia, HIV-associated dementia, Lewy body-related dementia, Down syndrome-related dementia, and vascular disease-related cognitive impairment.

In one embodiment, the cognitive impairment-related disease is mild cognitive impairment.

In one embodiment, the cognitive impairment-related disease is preclinical Alzheimer's disease.

In one embodiment, the cognitive impairment-related disease is Alzheimer's disease.

In one embodiment, the cognitive impairment-related disease is dementia of Alzheimer type.

In one embodiment, the cognitive impairment-related disease is prodromal AD.

In some other embodiments, the neurodegenerative disease is selected from at least one of: Parkinson's disease, Alzheimer's disease, Prion diseases, motor neuron disease (MND) such as amyotrophic lateral sclerosis (ALS), Huntington's disease (HD), spinocerebellar ataxia (SCA), spinal muscular atrophy (SMA), Friedreich's ataxia, Lewy body disease, epilepsy, encephalitis, hydrocephalus, stroke, chronic traumatic encephalopathy (CTE); synucleinopathies; tauopathies; spongiform encephalopathy; familial amyloid polyneuropathy; hereditary cerebral hemorrhage with amyloidosis-Dutch type; cerebral amyloid angiopathy; corticobasal degeneration; Pick's disease; progressive supranuclear palsy; Creutzfeldt-Jakob disease; Gerstmann syndrome; fatal familial insomnia; kuru; bovine spongiform encephalopathy; scrapie; chronic wasting disease; the Lewy body variant of Alzheimer's disease; diffuse Lewy body disease; dementia with Lewy bodies; multiple system atrophy; neurodegeneration with brain iron accumulation, type I; diffuse Lewy body disease; frontotemporal lobar degeneration; hereditary dentatorubral-pallidoluysian atrophy; Kennedy's disease; Alexander disease; Cockayne syndrome; and hereditary cerebral amyloid angiopathy, Icelandic type.

In some other embodiments, the neurodegenerative disease is selected from Parkinson's disease, Alzheimer's disease, Huntington's disease (HD), and motor neuron disease (MND) such as amyotrophic lateral sclerosis (ALS).

In some other embodiments, the neurodegenerative disease is Alzheimer's disease.

In some embodiments, the subject has one or more indications selected from: type I diabetes, type II diabetes, maturity-onset diabetes of the young (MODY), gestational diabetes, impaired glucose tolerance, abnormal fasting blood glucose level, hyperglycemia, postprandial hyperglycemia, overweight, obesity, hypertension, cardiovascular disease, insulin resistance and/or metabolic syndrome.

In some embodiments, the subject has one or more indications selected from: type I diabetes, type II diabetes, maturity-onset diabetes of the young (MODY), gestational diabetes, impaired glucose tolerance, abnormal fasting blood glucose level, hyperglycemia, postprandial hyperglycemia, insulin resistance and/or metabolic syndrome.

In some embodiments, the subject has one or more indications selected from: type I diabetes, type II diabetes and insulin resistance.

In some other embodiments, the subject has cognitive impairment or a neurodegenerative disease, and also has indications of insulin resistance or type II diabetes.

In some other embodiments, the present disclosure provides use of a pharmaceutical composition in the manufacture of a medicament for the prevention, alleviation or treatment of cognitive impairment or a neurodegenerative disease in a subject, or delaying the progression of cognitive impairment or a neurodegenerative disease in a subject, or reducing the risk of developing cognitive impairment or a neurodegenerative disease in a subject, wherein the pharmaceutical composition comprises a glucokinase activator or a prodrug thereof, or a pharmaceutically acceptable salt, an isotope-labeled form, a crystal form, a hydrate, a solvate, an enantiomer, or a diastereomer thereof; and optionally one or more pharmaceutically acceptable excipients.

In some embodiments, the present disclosure provides a method of preventing, alleviating or treating cognitive impairment or a neurodegenerative disease, or delaying the progression of cognitive impairment or a neurodegenerative disease, or reducing the risk of developing cognitive impairment or a neurodegenerative disease, comprising administering a glucokinase activator or a prodrug thereof, or a pharmaceutically acceptable salt, an isotope-labeled form, a crystal form, a hydrate, a solvate, a diastereomer or an enantiomer thereof to a subject in need.

In one embodiment, the present disclosure provides a method of preventing, alleviating, or treating cognitive impairment or a neurodegenerative disease, comprising administering a therapeutically effective amount of Dorzagliatin or a prodrug thereof, or a pharmaceutically acceptable salt thereof to a subject in need.

In one embodiment, the present disclosure provides a method of delaying the progression of cognitive impairment or a neurodegenerative disease, comprising administering a therapeutically effective amount of Dorzagliatin or a prodrug thereof, or a pharmaceutically acceptable salt thereof to a subject in need.

In one embodiment, the present disclosure provides a method of reducing the risk of developing cognitive impairment or a neurodegenerative disease, comprising administering a therapeutically effective amount of Dorzagliatin or a prodrug thereof, or a pharmaceutically acceptable salt thereof to a subject in need.

In some embodiments, the glucokinase activator is selected from at least one of: Dorzagliatin, AZD1656, piragliatin, AZD6370, GKA50, YH-GKA, PSN-010, LY2121260, Ganoderma lucidum polysaccharide B, eupatilin, glucolipsin A, glucolipsin B, GKM-001, TTP399, SY004, TMG-123, AM-9514, AMG-0696, AMG-1694, AMG-3969, LCZ-960, AZD-1092, AZD5658, ARRY-403, BMS-820132, GKM-002, LY-2608204, MK-0941, R-1511, RO-281675, ZYGK-1, OP-986CR, CM-3, DS-7309, LY-2599506, PF-04937319, PF04991532, TAK-329, PF-04991539, TAK-399, or a prodrug thereof, or a pharmaceutically acceptable salt, an isotope-labeled form, a crystal form, a hydrate, a solvate, a diastereomer or an enantiomer thereof.

In some other embodiments, the present disclosure relates to use of Dorzagliatin or a prodrug thereof, or a pharmaceutically acceptable salt, an isotope-labeled form, a crystal form, a hydrate, a solvate, a diastereomer or an enantiomer thereof, in the manufacture of a medicament for the prevention, alleviation or treatment of cognitive impairment or a neurodegenerative disease in a subject, or delaying the progression of cognitive impairment or a neurodegenerative disease in a subject, or reducing the risk of developing cognitive impairment or a neurodegenerative disease in a subject.

In some embodiments, the glucokinase activator is a compound represented by the following formula, or a pharmaceutically acceptable salt, an isotope-labeled form, a crystal form, a hydrate, a solvate, a diastereomer or an enantiomer thereof:

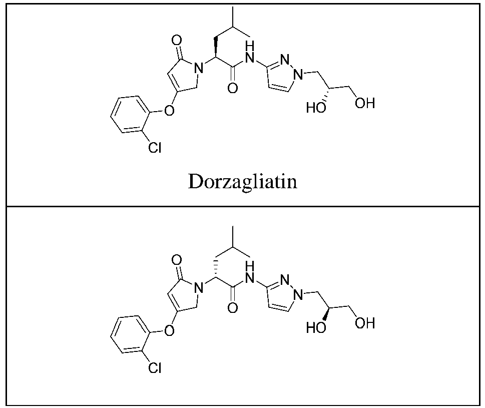

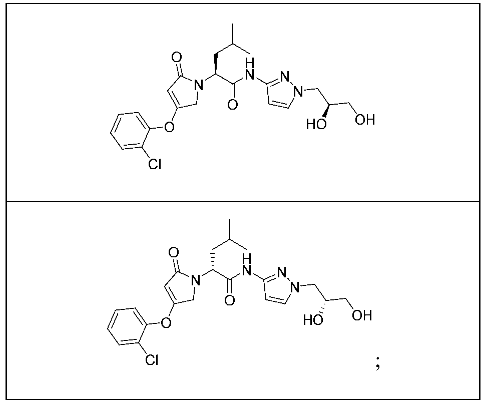

in some other embodiments, the glucokinase activator of the present disclosure is a compound represented by the following formula, or a pharmaceutically acceptable salt, an isotope-labeled form, a crystal form, a hydrate, a solvate, a diastereomer or an enantiomer thereof:

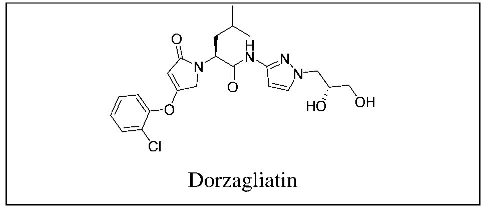

In another embodiment, the glucokinase activator is Dorzagliatin, or a pharmaceutically acceptable salt thereof.

In some other embodiments, the glucokinase activator is a prodrug of Dorzagliatin, or a pharmaceutically acceptable salt, an isotope-labeled form, a crystal form, a hydrate, a solvate, a diastereomer or an enantiomer thereof.

In another embodiment, the glucokinase activator is a prodrug of Dorzagliatin, or a pharmaceutically acceptable salt thereof.

In some other embodiments, the glucokinase activator is a prodrug of Dorzagliatin. The prodrug of Dorzagliatin is a compound disclosed in WO 2023/040937 A1, or a pharmaceutically acceptable salt, an isotope-labeled form, an enantiomer, or a diastereomer thereof, and the entire disclosure of the above patent application is incorporated herein by reference.

In some other embodiments, the prodrug of Dorzagliatin is a compound of formula (I), or an isotope-labeled form, an enantiomer, a diastereomer, or a pharmaceutically acceptable salt thereof: 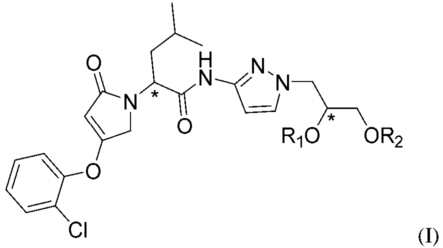 wherein
* indicates a chiral center,
R₁ is selected from H, -C(O)R₆, -C(O)OR₆, -C(O)NR₇R₈, -S(O)ₘR₆, -S(O)ₘOR₆ and -S(O)ₘNR₇R₈;
R₂ is selected from -C(O)R₃, -C(O)OR₃, -C(O)NR₄R₅, -S(O)ₘR₃, -S(O)ₘOR₃ and -S(O)ₘNR₄R₅;
or R₁ and R₂ are connected to form -CHR_{d}-, -SiR_{d}Rₑ₋, -C(O)-, -S(O)₁₋₂-, -P(O)OR_{d}-, or -CR_{d}Rₑ-CR_{d}Rₑ-;
R₃ is independently selected from H, C₁₋₆ alkyl, C₂₋₆ alkenyl, C₂₋₆ alkynyl, C₁₋₆ haloalkyl, C₃₋₇ cycloalkyl, 3- to 7-membered heterocyclyl, C₆₋₁₀ aryl and 5- to 10-membered heteroaryl, and R₃ is optionally substituted with 1, 2, 3, 4 or 5 R groups;
R₄ and R₅ are independently selected from H, C₁₋₆ alkyl, C₂₋₆ alkenyl, C₂₋₆ alkynyl and C₁₋₆ haloalkyl; or, R₄, R₅ and the N atom are taken together to form 3- to 7-membered heterocyclyl;
R₆ is independently selected from H, C₁₋₆ alkyl, C₂₋₆ alkenyl, C₂₋₆ alkynyl and C₁₋₆ haloalkyl;
R₇ and R₈ are independently selected from H, C₁₋₆ alkyl, C₂₋₆ alkenyl, C₂₋₆ alkynyl and C₁₋₆ haloalkyl; or, R₇, R₈ and the N atom are taken together to form 3- to 7-membered heterocyclyl;
R is independently selected from H, -L-halogen, -L-CN, -L-NO₂, -L-ORₐ, -L-SRₐ, -L-NR_{b}R_{c}, -L-C(O)ORₐ, -L-C(O)NR_{b}R_{c}, -L-S(O)ₘORₐ, -L-S(O)ₘNR_{b}R_{c}, C₁₋₆ alkyl, C₁₋₆ haloalkyl, C₂₋₆ alkenyl, C₂₋₆ alkynyl, C₁₋₆ haloalkyl, C₃₋₇ cycloalkyl, 3- to 7-membered heterocyclyl, C₆₋₁₀ aryl, 5- to 10-membered heteroaryl and a side chain of a natural amino acid;
wherein m = 1 or 2;
Rₐ is independently selected from H, C₁₋₆ alkyl, C₂₋₆ alkenyl, C₂₋₆ alkynyl, C₁₋₆ haloalkyl, C₃₋₇ cycloalkyl, 3- to 7-membered heterocyclyl, C₆₋₁₀ aryl and 5- to 10-membered heteroaryl;
R_{b} and R_{c} are independently selected from H, C₁₋₆ alkyl, C₂₋₆ alkenyl, C₂₋₆ alkynyl, C₁₋₆ haloalkyl, C₃₋₇ cycloalkyl, 3- to 7-membered heterocyclyl, C₆₋₁₀ aryl and 5- to 10-membered heteroaryl; or, R_{b}, R_{c} and the N atom are taken together to form 3- to 7-membered heterocyclyl;
R_{d} and Rₑ are independently selected from H, C₁₋₆ alkyl, C₂₋₆ alkenyl, C₂₋₆ alkynyl and C₁₋₆ haloalkyl, or, R_{d}, Rₑ and the C atom are taken together to form =O, =S, C₃₋₇ cycloalkyl or 3- to 7-membered heterocyclyl;
L is selected from chemical bond, -C₁₋₆ alkylene-, -C₂₋₆ alkenylene- and -C₂₋₆ alkynylene-.

In some embodiments, the prodrug of Dorzagliatin or a pharmaceutically acceptable salt thereof is selected from the compounds of the following structures:

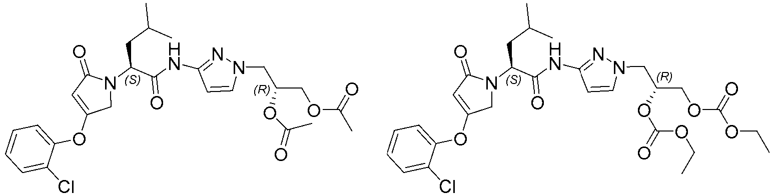

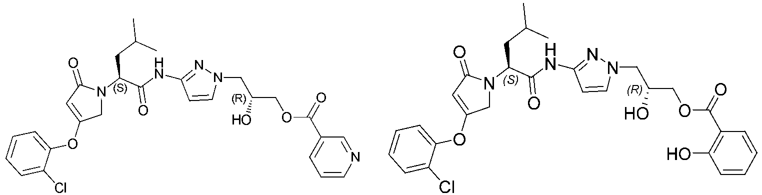

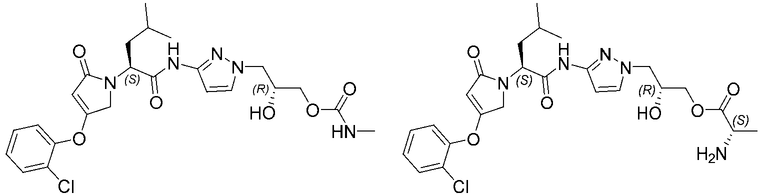

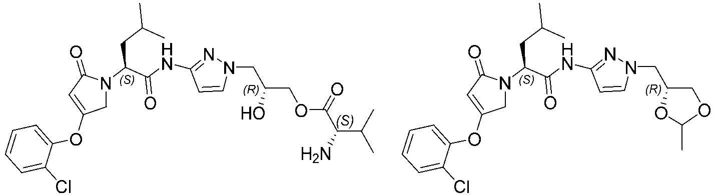

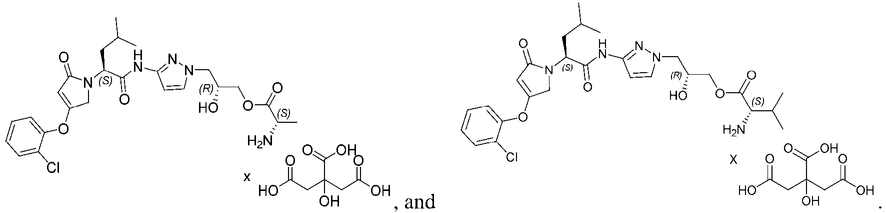

In some embodiments, the prodrug of Dorzagliatin is a compound of the following structure:

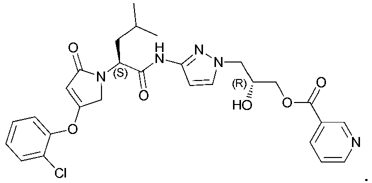

In some embodiments, symptoms of the cognitive impairment include decreased attention, decreased language ability, decreased spatial and temporal abilities, decreased reasoning ability, or decreased judgment.

For example, cognitive impairment includes the following symptoms: short-term or long-term memory function, learning ability, mental flexibility, attention, executive function, spatial working memory, digital working memory, picture recognition, word recognition, checking time, mood, energy, anger, hostility, confusion, and overall mood disorders.

In some embodiments, the cognitive impairment-related disease is selected from mild cognitive impairment (MCI), preclinical Alzheimer's disease, Alzheimer's disease, dementia of Alzheimer type, presenile dementia, early-onset Alzheimer's disease, prodromal AD, senile dementia, dementia with Lewy bodies (DLB), dementia of microinfarct, AIDS-related dementia, HIV-associated dementia, Lewy body-related dementia, Down syndrome-related dementia, frontotemporal dementia, Pick's disease, recent and short-term memory impairment, age-related cognitive impairment, age-related memory impairment, drug-related cognitive impairment, immunodeficiency syndrome-related cognitive impairment, vascular disease-related cognitive impairment, schizophrenia-related cognitive impairment, Parkinson's disease-related cognitive impairment, epilepsy-related cognitive impairment, depression-related cognitive impairment, bipolar disorder-related cognitive impairment, obsessive-compulsive disorder-related cognitive impairment, post-traumatic stress disorder, attention deficit disorder, attention-deficit hyperactivity disorder, and learning disabilities;

in some embodiments, the cognitive impairment-related disease is selected from mild cognitive impairment (MCI), preclinical Alzheimer's disease, Alzheimer's disease, dementia of Alzheimer type, presenile dementia, early-onset Alzheimer's disease, prodromal AD, senile dementia, dementia with Lewy bodies, dementia of microinfarct, AIDS-related dementia, HIV-associated dementia, Lewy body-related dementia, Down syndrome-related dementia, and vascular disease-related cognitive impairment.

In one embodiment, the cognitive impairment-related disease is mild cognitive impairment.

In one embodiment, the cognitive impairment-related disease is preclinical Alzheimer's disease.

In one embodiment, the cognitive impairment-related disease is Alzheimer's disease.

In one embodiment, the cognitive impairment-related disease is dementia of Alzheimer type.

In one embodiment, the cognitive impairment-related disease is prodromal AD.

In some other embodiments, range of the subject with cognitive impairment extends from mild cognitive impairment (MCI) to severe cognitive impairment.

In some other embodiments, the subject with cognitive impairment may have dementia of Alzheimer type, or mild Alzheimer's disease, or moderate Alzheimer's disease, or severe Alzheimer's disease.

In some other embodiments, the subject is a human being.

In some other embodiments, the neurodegenerative disease is selected from at least one of: Parkinson's disease, Alzheimer's disease, Prion diseases, motor neuron disease (MND) such as amyotrophic lateral sclerosis (ALS), Huntington's disease (HD), spinocerebellar ataxia (SCA), spinal muscular atrophy (SMA), Friedreich's ataxia, Lewy body disease, epilepsy, encephalitis, hydrocephalus, stroke, chronic traumatic encephalopathy (CTE); synucleinopathies; tauopathies; spongiform encephalopathy; familial amyloid polyneuropathy; hereditary cerebral hemorrhage with amyloidosis-Dutch type; cerebral amyloid angiopathy; corticobasal degeneration; Pick's disease; progressive supranuclear palsy; Creutzfeldt-Jakob disease; Gerstmann syndrome; fatal familial insomnia; kuru; bovine spongiform encephalopathy; scrapie; chronic wasting disease; the Lewy body variant of Alzheimer's disease; diffuse Lewy body disease; dementia with Lewy bodies; multiple system atrophy; neurodegeneration with brain iron accumulation, type I; diffuse Lewy body disease; frontotemporal lobar degeneration; hereditary dentatorubral-pallidoluysian atrophy; Kennedy's disease; Alexander disease; Cockayne syndrome; and hereditary cerebral amyloid angiopathy, Icelandic type.

In some other embodiments, the neurodegenerative disease is selected from Parkinson's disease, Alzheimer's disease, Huntington's disease (HD), and motor neuron disease (MND) such as amyotrophic lateral sclerosis (ALS).

In some other embodiments, the neurodegenerative disease is Alzheimer's disease.

In some other embodiments, the subject has one or more indications selected from: type I diabetes, type II diabetes, maturity-onset diabetes of the young (MODY), gestational diabetes, impaired glucose tolerance, abnormal fasting blood glucose level, hyperglycemia, postprandial hyperglycemia, overweight, obesity, hypertension, cardiovascular disease, insulin resistance and/or metabolic syndrome.

In some embodiments, the subject has one or more indications selected from: type I diabetes, type II diabetes, maturity-onset diabetes of the young (MODY), gestational diabetes, impaired glucose tolerance, abnormal fasting blood glucose level, hyperglycemia, postprandial hyperglycemia, insulin resistance and/or metabolic syndrome.

In some embodiments, the subject has one or more indications selected from: type I diabetes, type II diabetes and insulin resistance.

In some other embodiments, the subject has cognitive impairment or a neurodegenerative disease, and also has indications of insulin resistance or type II diabetes.

In some other embodiments, the glucokinase activator may be administered in the form of a pharmaceutical composition. The above pharmaceutical composition comprises a glucokinase activator or a prodrug thereof, or an isotope-labeled form, a crystal form, a hydrate, a solvate, a diastereomer, or an enantiomer thereof; and optionally one or more pharmaceutically acceptable excipients.

In some other embodiments, the dosage of the glucokinase activator is from 0.01 mg to 500 mg, alternatively from 0.01 mg to 200 mg, yet alternatively from 0.1 mg to 150 mg, such as 1 mg, 10 mg, 20 mg, 30 mg, 40 mg, 50 mg, 60 mg, 70 mg, 80 mg, 90 mg, 100 mg, 110 mg, 120 mg, 130 mg, 140 mg, or 150 mg.

In some other embodiments, the glucokinase activator is administered orally, parenterally, by inhalation, or topically, alternatively orally.

In some other embodiments, the glucokinase activator is administered for 3 months, 4 months, 5 months, 6 months, 1 year, 2 years, 3 years, 5 years, 6 years, 7 years, 8 years, 9 years, or 10 years.

In some other embodiments, the glucokinase activator is administered once daily, twice daily, three times daily, or four times daily, alternatively twice daily.

In some other embodiments, the glucokinase activator is administered before or after a meal, alternatively after a meal.

In some embodiments, the present disclosure provides a glucokinase activator or a prodrug thereof, or a pharmaceutically acceptable salt, an isotope-labeled form, a crystal form, a hydrate, a solvate, a diastereomer or an enantiomer thereof, for use in the prevention, alleviation or treatment of cognitive impairment or a neurodegenerative disease, or delaying the progression of cognitive impairment or a neurodegenerative disease, or reducing the risk of developing cognitive impairment or a neurodegenerative disease.

In some embodiments, the glucokinase activator of the present disclosure is selected from at least one of: Dorzagliatin, AZD1656, piragliatin, AZD6370, GKA50, YH-GKA, PSN-010, LY2121260, Ganoderma lucidum polysaccharide B, eupatilin, glucolipsin A, glucolipsin B, GKM-001, TTP399, SY004, TMG-123, AM-9514, AMG-0696, AMG-1694, AMG-3969, LCZ-960, AZD-1092, AZD5658, ARRY-403, BMS-820132, GKM-002, LY-2608204, MK-0941, R-1511 RO-281675, ZYGK-1, OP-986CR, CM-3, DS-7309, LY-2599506, PF-04937319, PF04991532, TAK-329, PF-04991539, TAK-399, or a prodrug thereof, or a pharmaceutically acceptable salt, an isotope-labeled form, a crystal form, a hydrate, a solvate, a diastereomer or an enantiomer thereof.

In some other embodiments, the present disclosure relates to use of Dorzagliatin or a prodrug thereof, or a pharmaceutically acceptable salt, an isotope-labeled form, a crystal form, a hydrate, a solvate, a diastereomer or an enantiomer thereof, in the manufacture of a medicament for the prevention, alleviation or treatment of cognitive impairment or a neurodegenerative disease in a subject, or delaying the progression of cognitive impairment or a neurodegenerative disease in a subject, or reducing the risk of developing cognitive impairment or a neurodegenerative disease in a subject.

In some other embodiments, the glucokinase activator of the present disclosure is a compound represented by the following formula, or a pharmaceutically acceptable salt, an isotope-labeled form, a crystal form, a hydrate, a solvate, a diastereomer or an enantiomer thereof:

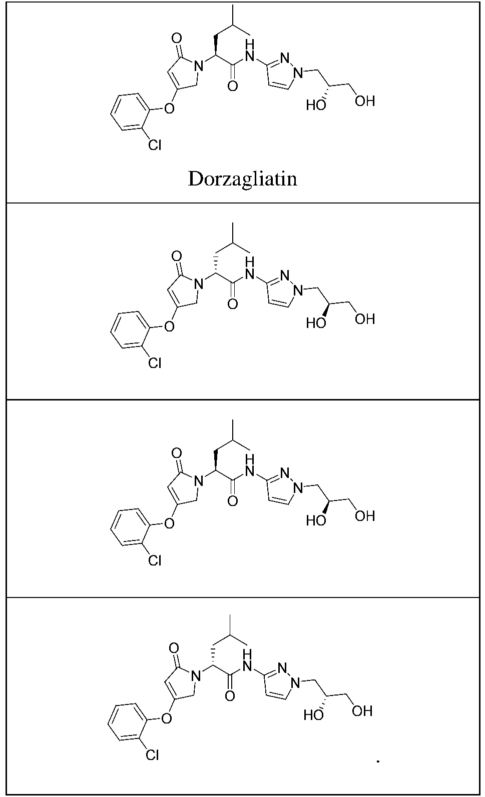

Alternatively, the glucokinase activator of the present disclosure is a compound represented by the following formula, or a pharmaceutically acceptable salt, an isotope-labeled form, a crystal form, a hydrate, or a solvate thereof:

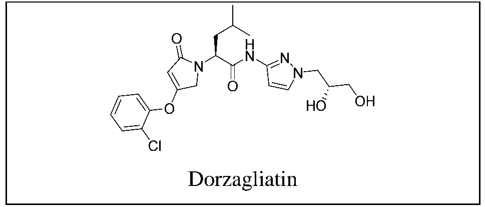

Alternatively, the glucokinase activator of the present disclosure is Dorzagliatin, or a pharmaceutically acceptable salt thereof.

In some other embodiments, the glucokinase activator of the present disclosure is a prodrug of Dorzagliatin, or a pharmaceutically acceptable salt, an isotope-labeled form, a crystal form, a hydrate, a solvate, a diastereomer or an enantiomer thereof.

Alternatively, the glucokinase activator of the present disclosure is a prodrug of Dorzagliatin, or a pharmaceutically acceptable salt thereof.

In some other embodiments, the prodrug of Dorzagliatin is a compound disclosed in WO 2023/040937 A1, or a pharmaceutically acceptable salt, an isotope-labeled form, an enantiomer or a diastereomer thereof, and the entire disclosure of the above patent application is incorporated herein by reference.

In some other embodiments, the prodrug of Dorzagliatin is a compound of formula (I), or an isotope-labeled form, an enantiomer, a diastereomer, or a pharmaceutically acceptable salt thereof: 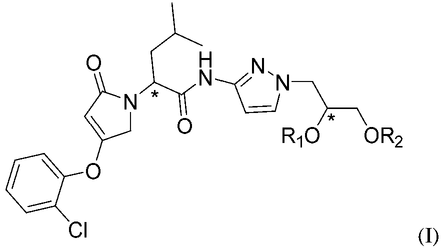 wherein
* indicates a chiral center,
R₁ is selected from H, -C(O)R₆, -C(O)OR₆, -C(O)NR₇R₈, -S(O)ₘR₆, -S(O)ₘOR₆ and -S(O)ₘNR₇R₈;
R₂ is selected from -C(O)R₃, -C(O)OR₃, -C(O)NR₄R₅, -S(O)ₘR₃, -S(O)ₘOR₃ and -S(O)ₘNR₄R₅;
or R₁ and R₂ are connected to form -CHR_{d}-, -SiR_{d}Rₑ₋, -C(O)-, -S(O)ᵢ₋₂-, -P(O)OR_{d}-, or -CR_{d}Rₑ-CR_{d}Rₑ-;
R₃ is independently selected from H, C₁₋₆ alkyl, C₂₋₆ alkenyl, C₂₋₆ alkynyl, C₁₋₆ haloalkyl, C₃₋₇ cycloalkyl, 3- to 7-membered heterocyclyl, C₆₋₁₀ aryl and 5- to 10-membered heteroaryl, and R₃ is optionally substituted with 1, 2, 3, 4 or 5 R groups;
R₄ and R₅ are independently selected from H, C₁₋₆ alkyl, C₂₋₆ alkenyl, C₂₋₆ alkynyl and C₁₋₆ haloalkyl; or, R₄, R₅ and the N atom are taken together to form 3- to 7-membered heterocyclyl;
R₆ is independently selected from H, C₁₋₆ alkyl, C₂₋₆ alkenyl, C₂₋₆ alkynyl and C₁₋₆ haloalkyl;
R₇ and R₈ are independently selected from H, C₁₋₆ alkyl, C₂₋₆ alkenyl, C₂₋₆ alkynyl and C₁₋₆ haloalkyl; or, R₇, R₈ and the N atom are taken together to form 3- to 7-membered heterocyclyl;
R is independently selected from H, -L-halogen, -L-CN, -L-NO₂, -L-ORₐ, -L-SRₐ, -L-NR_{b}R_{c}, -L-C(O)ORₐ, -L-C(O)NR_{b}R_{c}, -L-S(O)ₘORₐ, -L-S(O)ₘNR_{b}R_{c}, C₁₋₆ alkyl, C₁₋₆ haloalkyl, C₂₋₆ alkenyl, C₂₋₆ alkynyl, C₁₋₆ haloalkyl, C₃₋₇ cycloalkyl, 3- to 7-membered heterocyclyl, C₆₋₁₀ aryl, 5- to 10-membered heteroaryl and a side chain of a natural amino acid;
wherein m = 1 or 2;
Rₐ is independently selected from H, C₁₋₆ alkyl, C₂₋₆ alkenyl, C₂₋₆ alkynyl, C₁₋₆ haloalkyl, C₃₋₇ cycloalkyl, 3- to 7-membered heterocyclyl, C₆₋₁₀ aryl and 5- to 10-membered heteroaryl;
R_{b} and R_{c} are independently selected from H, C₁₋₆ alkyl, C₂₋₆ alkenyl, C₂₋₆ alkynyl, C₁₋₆ haloalkyl, C₃₋₇ cycloalkyl, 3- to 7-membered heterocyclyl, C₆₋₁₀ aryl and 5- to 10-membered heteroaryl; or, R_{b}, R_{c} and the N atom are taken together to form 3- to 7-membered heterocyclyl;
R_{d} and Rₑ are independently selected from H, C₁₋₆ alkyl, C₂₋₆ alkenyl, C₂₋₆ alkynyl and C₁₋₆ haloalkyl, or, R_{d}, Rₑ and the C atom are taken together to form =O, =S, C₃₋₇ cycloalkyl or 3- to 7-membered heterocyclyl;
L is selected from chemical bond, -C₁₋₆ alkylene-, -C₂₋₆ alkenylene- and -C₂₋₆ alkynylene-.

In some embodiments, the prodrug of Dorzagliatin or a pharmaceutically acceptable salt thereof is selected from the compounds of the following structures:

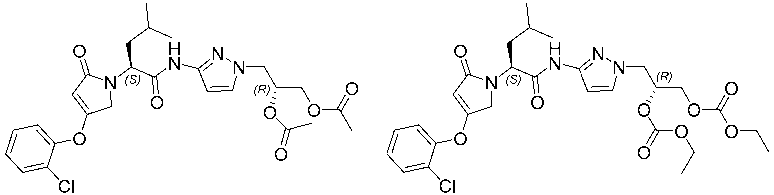

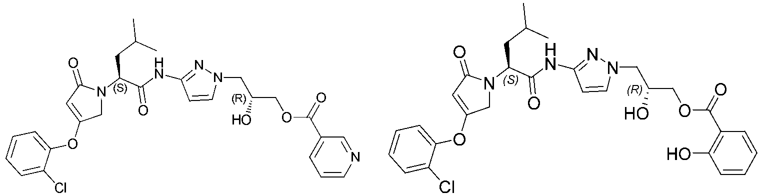

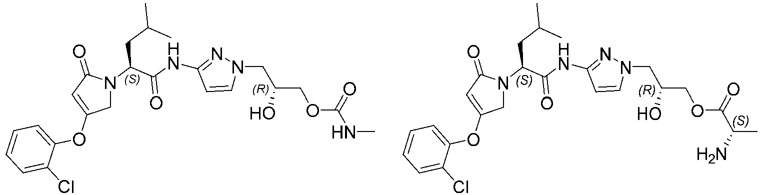

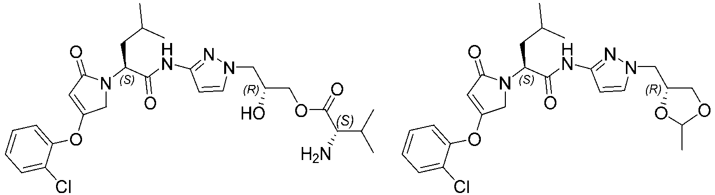

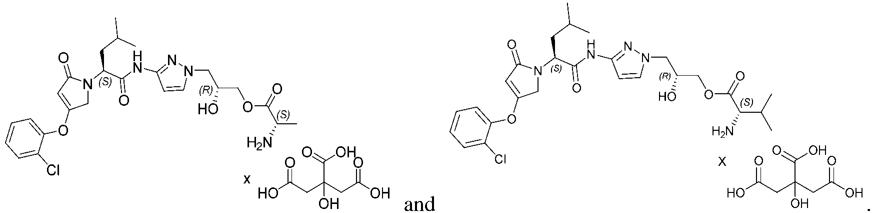

In some other embodiments, the prodrug of Dorzagliatin is a compound of the following structure:

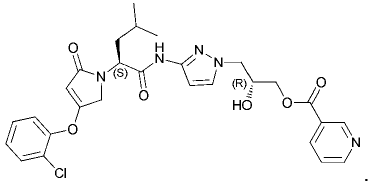

In some embodiments, symptoms of cognitive impairment include decreased attention, decreased language ability, decreased spatial and temporal abilities, decreased reasoning ability, or decreased judgment.

For example, cognitive impairment includes the following symptoms: short-term or long-term memory function, learning ability, mental flexibility, attention, executive function, spatial working memory, digital working memory, picture recognition, word recognition, checking time, mood, energy, anger, hostility, confusion, and overall mood disorders.

In some embodiments, the glucokinase activator is for use in the prevention, alleviation or treatment of a cognitive impairment-related disease, or delaying the progression of a cognitive impairment-related disease, or reducing the risk of developing a cognitive impairment-related disease.

In some embodiments, the cognitive impairment-related disease is selected from mild cognitive impairment (MCI), preclinical Alzheimer's disease, Alzheimer's disease, dementia of Alzheimer type, presenile dementia, early-onset Alzheimer's disease, prodromal AD, senile dementia, dementia with Lewy bodies (DLB), dementia of microinfarct, AIDS-related dementia, HIV-associated dementia, Lewy body-related dementia, Down syndrome-related dementia, frontotemporal dementia, Pick's disease, recent and short-term memory impairment, age-related cognitive impairment, age-related memory impairment, drug-related cognitive impairment, immunodeficiency syndrome-related cognitive impairment, vascular disease-related cognitive impairment, schizophrenia-related cognitive impairment, Parkinson's disease-related cognitive impairment, epilepsy-related cognitive impairment, depression-related cognitive impairment, bipolar disorder-related cognitive impairment, obsessive-compulsive disorder-related cognitive impairment, post-traumatic stress disorder, attention deficit disorder, attention-deficit hyperactivity disorder, and learning disabilities.

In some embodiments, the cognitive impairment-related disease is selected from mild cognitive impairment (MCI), preclinical Alzheimer's disease, Alzheimer's disease, dementia of Alzheimer type, presenile dementia, early-onset Alzheimer's disease, prodromal AD, senile dementia, dementia with Lewy bodies, dementia of microinfarct, AIDS-related dementia, HIV-associated dementia, Lewy body-related dementia, Down syndrome-related dementia, and vascular disease-related cognitive impairment.

In one embodiment, the cognitive impairment-related disease is mild cognitive impairment.

In one embodiment, the cognitive impairment-related disease is preclinical Alzheimer's disease.

In one embodiment, the cognitive impairment-related disease is Alzheimer's disease.

In one embodiment, the cognitive impairment-related disease is dementia of Alzheimer type.

In one embodiment, the cognitive impairment-related disease is prodromal AD.

In some other embodiments, the neurodegenerative disease is selected from at least one of: Parkinson's disease, Alzheimer's disease, Prion diseases, motor neuron disease (MND) such as amyotrophic lateral sclerosis (ALS), Huntington's disease (HD), spinocerebellar ataxia (SCA), spinal muscular atrophy (SMA), Friedreich's ataxia, Lewy body disease, epilepsy, encephalitis, hydrocephalus, stroke, chronic traumatic encephalopathy (CTE); synucleinopathies; tauopathies; spongiform encephalopathy; familial amyloid polyneuropathy; hereditary cerebral hemorrhage with amyloidosis-Dutch type; cerebral amyloid angiopathy; corticobasal degeneration; Pick's disease; progressive supranuclear palsy; Creutzfeldt-Jakob disease; Gerstmann syndrome; fatal familial insomnia; kuru; bovine spongiform encephalopathy; scrapie; chronic wasting disease; the Lewy body variant of Alzheimer's disease; diffuse Lewy body disease; dementia with Lewy bodies; multiple system atrophy; neurodegeneration with brain iron accumulation, type I; diffuse Lewy body disease; frontotemporal lobar degeneration; hereditary dentatorubral-pallidoluysian atrophy; Kennedy's disease; Alexander disease; Cockayne syndrome; and hereditary cerebral amyloid angiopathy, Icelandic type.

In some other embodiments, the neurodegenerative disease is selected from Parkinson's disease, Alzheimer's disease, Huntington's disease (HD), and motor neuron disease (MND) such as amyotrophic lateral sclerosis (ALS).

In some other embodiments, the neurodegenerative disease is Alzheimer's disease.

In some embodiments, the subject has one or more indications selected from: type I diabetes, type II diabetes, maturity-onset diabetes of the young (MODY), gestational diabetes, impaired glucose tolerance, abnormal fasting blood glucose level, hyperglycemia, postprandial hyperglycemia, overweight, obesity, hypertension, cardiovascular disease, insulin resistance and/or metabolic syndrome.

In some embodiments, the subject has one or more indications selected from: type I diabetes, type II diabetes, maturity-onset diabetes of the young (MODY), gestational diabetes, impaired glucose tolerance, abnormal fasting blood glucose level, hyperglycemia, postprandial hyperglycemia, insulin resistance and/or metabolic syndrome.

In some embodiments, the subject has one or more indications selected from: type I diabetes, type II diabetes and insulin resistance.

In some other embodiments, the subject has cognitive impairment or a neurodegenerative disease, and also has indications of insulin resistance or type II diabetes.

The glucokinase activator of the present disclosure (e.g., Dorzagliatin) may include one or more asymmetric centers, and thus may exist in a variety of stereoisomeric forms, for example, enantiomers and/or diastereomers. For example, the compounds of the present disclosure may be in the form of an individual enantiomer, diastereomer or geometric isomer (e.g., cis- and trans-isomers), or may be in the form of a mixture of stereoisomers, including racemic mixture and a mixture enriched in one or more stereoisomers. The isomers can be separated from the mixture by the methods known to those skilled in the art, including chiral high pressure liquid chromatography (HPLC) and the formation and crystallization of chiral salts; or alternative isomers can be prepared by asymmetric synthesis.

It will be understood by those skilled in the art that the organic compounds can form complexes with solvents in which they are reacted or from which they are precipitated or crystallized. These complexes are known as "solvates". Where the solvent is water, the complex is known as "hydrate". The present disclosure encompasses all solvates of the compound of the present disclosure.

The term "solvate" refers to forms of a compound or a salt thereof, which are associated with a solvent, usually by a solvolysis reaction. This physical association may include hydrogen bonding. Conventional solvents include water, methanol, ethanol, acetic acid, DMSO, THF, diethyl ether, etc. The compounds described herein can be prepared, for example, in crystalline form, and can be solvated. Suitable solvates include pharmaceutically acceptable solvates and further include both stoichiometric solvates and non-stoichiometric solvates. In some cases, the solvates will be capable of isolation, for example, when one or more solvent molecules are incorporated into the crystal lattice of a crystalline solid. "Solvate" includes both solution-phase and isolatable solvates. Representative solvates include hydrates, ethanolates and methanolates.

The term "hydrate" refers to a compound that is associated with water. Generally, the number of water molecules contained in a hydrate of a compound is in a definite ratio to the number of the compound molecules in the hydrate. Therefore, hydrates of a compound can be represented, for example, by a general formula R·x H₂O, wherein R is the compound, and x is a number greater than 0. Given compounds can form more than one type of hydrates, including, for example, monohydrates (x is 1), lower hydrates (x is a number greater than 0 and smaller than 1, for example, hemihydrates (R·0.5 H₂O)) and polyhydrates (x is a number greater than 1, for example, dihydrates (R·2 H₂O) and hexahydrates (R·6 H₂O)).

The present disclosure also comprises compounds that are labeled with isotopes (isotopic variants), which are equivalent to those described in formula (I), but one or more atoms are replaced by atoms having an atom mass or mass number that are different from that of atoms that are common in nature. Examples of isotopes which may be introduced into the compounds of the disclosure include isotopes of hydrogen, carbon, nitrogen, oxygen, phosphorus, sulfur, fluorine and chlorine, such as ²H, ³H, ¹³C, ¹¹C, ¹⁴C, ¹⁵N, ¹⁸O, ¹⁷O, ³¹P, ³²P, ³⁵S, ¹⁸F and ³⁶Cl, respectively. Compounds of the present disclosure that comprise the above isotopes and/or other isotopes of other atoms, prodrugs thereof and pharmaceutically acceptable salts of said compounds or prodrugs all are within the scope of the present disclosure. Certain isotope-labeled compounds of the present disclosure, such as those incorporating radioactive isotopes (e.g., ³H and ¹⁴C), can be used for the measurement of the distribution of drug and/or substrate in tissue. Tritium, which is ³H and carbon-14, which is ¹⁴C isotope, are yet alternative, because they are easy to prepare and detect. Furthermore, replaced by heavier isotopes, such as deuterium, which is ²H, may provide therapeutic benefits due to the higher metabolic stability, such as prolonging the half-life in vivo or decreasing the dosage requirements, and thus may be alternative in some cases. Isotope-labeled compounds of formula (I) of the present disclosure and prodrugs thereof can be prepared generally by using readily available isotope-labeled reagents to replace non-isotope-labeled reagents in the following schemes and/or the procedures disclosed in the examples and preparation examples.

In addition, prodrugs are also included within the context of the present disclosure. The term "prodrug" as used herein refers to a compound that releases an active drug to exert the medical effects in vivo by an enzymatic or a non-enzymatic conversion. Pharmaceutically acceptable prodrugs are exemplarily described in T. Higuchi and V. Stella, Prodrugs as Novel Delivery Systems, A.C.S. Symposium Series, Vol. 14, Edward B. Roche, ed., Bioreversible Carriers in Drug Design, American Pharmaceutical Association and Pergamon Press, 1987, and D. Fleisher, S. Ramon and H. Barbra "Improved oral drug delivery: solubility limitations overcome by the use of prodrugs", Advanced Drug Delivery Reviews (1996) 19(2) 115-130, each of which is incorporated herein by reference.

### Administration

The glucokinase activator (hereinafter referred to as the compound) provided by the present disclosure can be administered by a variety of routes including, but not limited to, oral administration, parenteral administration, inhalation administration, topical administration, rectal administration, nasal administration, oromucosal administration, vaginal administration, administration by implant or other means of administration. For example, parenteral administration as used herein includes subcutaneous administration, intradermal administration, intravenous administration, intramuscular administration, intra-articular administration, intraarterial administration, intrasynovial administration, intrasternal administration, intrathecal administration, intralesional administration, and intracranial injection or infusion techniques.

Generally, the compounds provided herein are administered in an effective amount. The amount of the compound actually administered may be determined by a physician, in the light of the relevant circumstances, including the condition to be treated, the chosen route of administration, the actual compound administered, the age, weight, and response of the individual patient, the severity of the patient's symptoms, and the like.

When used to prevent the condition disclosed herein, the compounds provided herein will be administered to a subject at risk for developing the condition, typically on the advice and under the supervision of a physician, at the dosage levels described above. Subjects at risk for developing a particular condition generally include those that have a family history of the condition, or those who have been identified by genetic testing or screening to be particularly susceptible to developing the condition.

The compounds provided herein or pharmaceutical compositions thereof can also be administered chronically ("chronic administration"). Chronic administration refers to the administration of a compound or pharmaceutical composition thereof for a long period of time, for example, 1 week, 2 weeks, 3 weeks, 1 month, 2 months, 3 months, 4 months, 5 months, 6 months, 1 year, 2 years, 3 years, 4 years, 5 years, 6 years, 7 years, 8 years, 9 years or 10 years, etc., or can be continuously administered indefinitely, for example, for the rest of the subject's life. In some embodiments, the chronic administration is intended to provide a constant level of the said compound in the blood over a long period of time, for example, within the therapeutic window.

The pharmaceutical compostions of the present disclosure may be further delivered using a variety of dosing methods. For example, in certain embodiments, the pharmaceutical composition may be given as a bolus, e.g., in order to raise the concentration of the compound in the blood to an effective level. The bolus dose depends on the systemic levels of the active ingredient desired throughout the body, e.g., an intramuscular or subcutaneous bolus dose allows a slow release of the active ingredient, while a bolus delivered directly to the veins (e.g., through an IV drip) allows a much faster delivery which quickly raises the concentration of the active ingredient in the blood to an effective level. In other embodiments, the pharmaceutical composition may be administered as a continuous infusion, e.g., by IV drip, to provide maintenance of a steady-state concentration of the active ingredient in the subject's body. Furthermore, in still yet other embodiments, the pharmaceutical composition may be administered as first as a bolus dose, followed by continuous infusion.

The compositions for oral administration can take the form of bulk liquid solutions or suspensions, or bulk powders. More commonly, however, the compositions are presented in unit dosage forms to facilitate accurate dosing. The term "unit dosage forms" refers to physically discrete units suitable as unitary dosages for human subjects and other mammals, each unit containing a predetermined quantity of active material suitable for producing the desired therapeutic effect, in association with a suitable pharmaceutical excipient. Typical unit dosage forms include prefilled, premeasured ampules or syringes of the liquid compositions or pills, tablets, capsules or the like in the case of solid compositions. In such compositions, the said compound generally will be the minor component (about 0.1 to about 50% by weight, or alternatively about 1 to about 40% by weight), with the remainder being various carriers or excipients and processing aids useful for forming the desired dosing form.

For oral dosage, a representative scheme is one to five, especially two to four, and typically three oral doses per day. Using these dosing patterns, each dose provides from about 0.01 to about 20 mg/kg of the compound of the present disclosure, with alternative doses each providing from about 0.1 to about 10 mg/kg, and especially from about 1 to about 5 mg/kg.

The effective amount of the compound of the present disclosure is generally at an average daily dosage of from 0.01 mg to 20 mg of the compound per kilogram of patient's body weight, alternatively from 0.01 mg to 10 mg of the compound per kilogram of patient's body weight, administered in a single or multiple doses. Generally, the compound of the present disclosure may be administered to a patient in need of such treatment in a daily dosage range from about 0.01 mg to about 1000 mg per patient, alternatively from 0.01 mg to 500 mg per patient. For example, the daily dosage for each patient may be 10, 20, 30, 40, 50, 60, 70, 80, 90, 100, 150, 200, 250, 300, 350, 400 or 500 mg. It can be administered one or more times, for example, one time, two times, three times or four times, daily, weekly (or at intervals of several days) or on an intermittent schedule. For example, the compound can be administered one or more times per day on a weekly basis (e.g., every Monday), for an indefinite period or for several weeks, such as 4-10 weeks. Alternatively, the compound may be administered daily for several days (e.g., 2-10 days) and then the compound is not administered for several days (e.g., 1-30 days), and the cycle may be repeated indefinitely or for a given number of times, e.g., 4-10 cycles. For example, the compound of the present disclosure may be administered daily for 5 days, followed by a 9-day break, then administered daily for 5 days, followed by a 9-day break, and so on, repeating this cycle indefinitely or a total of 4-10 times.

Transdermal doses are generally selected to provide similar or lower blood levels than are achieved using injection doses, generally in an amount ranging from about 0.01 to about 20% by weight, alternatively from about 0.1 to about 20% by weight, alternatively from about 0.1 to about 10% by weight, and still alternatively from about 0.5 to about 15% by weight.

The injection dosage level ranges from about 0.1 mg/kg/hr to at least 10 mg/kg/hr, for from about 1 to about 120 hours, especially from 24 to 96 hours. In order to achieve a sufficient level of steady state, a preloading bolus of from about 0.1 mg/kg to about 10 mg/kg or more can also be administered. For human patients of 40 to 80 kg, the maximum total dosage should not exceed approximately 2 g/day.

Liquid forms suitable for oral administration may include a suitable aqueous or nonaqueous vehicle, as well as buffers, suspending agents, dispensing agents, colorants, flavors and the like. Solid forms may include, for example, any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate; a glidant such as colloidal silicon dioxide; a sweetening agent such as sucrose or saccharin; or a flavoring agent such as peppermint, methyl salicylate, or orange flavoring.

Injectable compositions are typically based upon injectable sterile saline or phosphate-buffered saline or other injectable excipients known in the art. As previously mentioned, in such compositions, the active compounds will typically be the minor component, often from about 0.05 to 10% by weight, with the remainder being injectable excipients, etc.

Transdermal compositions are typically formulated as a topical ointment or cream containing the active ingredient(s). When formulated as an ointment, the active ingredients will typically be combined with either a paraffinic or a water-miscible ointment base. Alternatively, the active ingredients may be formulated in a cream with, for example an oil-in-water cream base. Such transdermal formulations are well-known in the art and generally include additional ingredients to enhance a stable dermal penetration of the active ingredients or formulation. All such known transdermal formulations and ingredients are included within the scope provided herein.

The compounds provided herein can also be administered by a transdermal device. Accordingly, transdermal drug delivery can be achieved using a patch with a reservoir, a porous membrane, or various solid matrix components.

The above-described components for orally administrable, injectable or topically administrable compositions are merely representative. Other materials as well as processing techniques and the like are set forth in Part 8 of Remington's Pharmaceutical Sciences, 17th edition, 1985, Mack Publishing Company, Easton, Pennsylvania, which is incorporated herein by reference.

The compounds of the present disclosure can also be administered in sustained release forms or from sustained release drug delivery systems. A description of representative sustained release materials can be found in Remington's Pharmaceutical Sciences.

The present disclosure also relates to the pharmaceutically acceptable formulations of a compound of the present disclosure. In one embodiment, the formulation comprises water. In another embodiment, the formulation comprises a cyclodextrin derivative. The most common cyclodextrins are α-, β- and γ-cyclodextrins consisting of 6, 7 and 8 α-1,4-linked glucose units, respectively, optionally comprising one or more substituents on the linked sugar moieties, which include, but are not limited to, methylated, hydroxyalkylated, acylated, and sulfoalkylether substitution. In certain embodiments, the cyclodextrin is a sulfoalkyl ether β-cyclodextrin, e.g., sulfobutyl ether β-cyclodextrin, also known as Captisol. See, for example, U.S.5,376,645. In certain embodiments, the formulation comprises hexapropyl-β-cyclodextrin (e.g., 10-50% in water).

Any technical solution in any one of the above specific embodiments, or any combination thereof, may be combined with any technical solution in other specific embodiments or any combination thereof. The present disclosure is intended to include all combinations of such technical solutions, which are not exhaustively listed here to save space.

### Examples

The materials or reagents used herein are commercially available or prepared by synthetic methods commonly known in the art.

The following examples further describe and illustrate embodiments that are within the scope of the present disclosure. However, the present disclosure is not limited to the examples, and any modifications and replacements made on the technical basis of the present disclosure fall within the protective scope of the present disclosure.

The prodrug of Dorzagliatin was prepared and tested in accordance with the prior art in WO 2023/040937 A1.

### Example 1: Dorzagliatin regulated the blood glucose homeostasis and delayed the progression of cognitive impairment in GK rats.

Goto-Kakizaki (GK) rats are a non-obese, spontaneously occurring type 2 diabetes rat model obtained through repeated inbreeding of Wistar rats with glucose tolerance at the upper limit over several generations. GK rats have normal blood glucose levels at birth, but gradually develop overt diabetes with age, exhibiting symptoms such as mild fasting hyperglycemia, significant postprandial hyperglycemia, and insulin resistance. Previous studies have shown that GK rats exhibit age-related metabolic complications with the increasing age, gradually developing a memory impairment phenotype and showing impaired development of hippocampal synapses, including measurable cognitive decline and decreased expression of synapse-related proteins. The hippocampus is a key functional brain region regulating learning and memory abilities. Due to the presence of progressive diabetes symptoms, typical neurodegenerative diseases, and memory deficits, GK rats can be used to evaluate the effects of a drug on preventing memory decline.

GK rats were randomly divided into the following treatment groups: a GK rat-solvent treatment group (n=9) and a GK rat-Dorzagliatin (8 mg/kg) treatment group (n=8). Age-matched Wistar rats were used as the normal control group, i.e., the Wistar rat-solvent group (n=9). The treatment began at six weeks of age. The rats were administered either the solvent or Dorzagliatin by gavage twice daily, morning and evening. Peripheral blood was collected multiple times from the animals throughout the growth cycle, and the fasting blood glucose levels of the animals were tested (the fasting blood glucose levels of the three groups of rats were tested and analyzed at weeks 6, 8, 12, 16, 20, 29, and 31).

The Morris water maze test is a commonly used behavioral method to assess the spatial memory, working memory, and spatial discrimination abilities of rodents. By using the method of searching for a target in an opaque pool by animals, observing and recording the time required for animals to search for a hidden underwater platform, as well as the strategies they employed and their swimming trajectories, we can analyze and infer the learning, memory, spatial orientation, and cognitive abilities of the animals.

The Morris water maze test was performed at approximately 27 weeks of treatment, i.e. when the animals were 33 weeks old.

Preparation of the experimental environment: The laboratory environment was kept quiet. A circular maze/pool (135 cm in diameter, 45 cm in height) was set up. Visual references (spatial cues) were placed on the walls of the pool and around the pool for the animals to learn from. The water temperature in the pool was equilibrated to room temperature (22 °C). The pool area was subdivided into four virtual quadrants (northeast, northwest, southeast, and southwest), with the northeast quadrant defined as the target quadrant, containing a hidden escape platform. During the test, the latent period of escape, swimming path (distance), speed, and quadrant preference of a rat were recorded using the ANY-maze system.

Training phase: The experimental rats were placed at the starting point of the water maze and allowed to swim freely, searching for the hidden escape platform. Once the rats found the platform, they could rest on the platform for 15 seconds. Each training trial lasted 60 seconds. If the rats could not find the platform within 60 seconds, they were guided to the platform and stayed on the platform for 15 seconds to experience the feeling of being on the platform. This process improved learning performance from the second attempt onwards. Four trials were performed daily during the training period, with a 30-minute interval between each trial. The location of the hidden platform remained unchanged throughout the training.

Spatial memory test (probe test): The spatial memory test was performed 24 hours after the end of the training phase. During the test phase, the hidden platform was removed from the water maze, and then the rats were placed at the starting point and allowed to swim freely for 1 minute. The time spent in the original platform quadrant and the swimming distances were recorded, and the data of each group of animals were statistically analyzed.

### Results

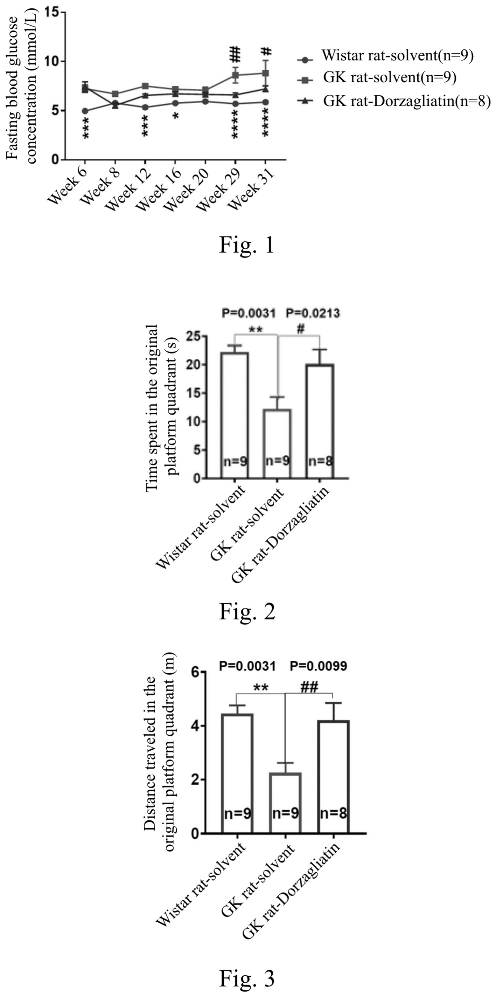

The data in Fig. 1 show that, with increasing age, the fasting blood glucose levels of the Wistar rat-solvent group remained relatively stable, while the blood glucose levels of the GK rat-solvent group gradually increased and were significantly higher than those of the Wistar rat-solvent group (*P<0.05, ***P<0.001, ****P<0.0001; two-way ANOVA, Dunnett's multiple comparison test). The blood glucose levels of the GK rat-Dorzagliatin group were significantly lower than those of the GK rat-solvent group (#P<0.05, ##P<0.01; two-way ANOVA, Dunnett's multiple comparison test).

These data indicate that fasting blood glucose levels of the GK rat-solvent group gradually increased with age, while the long-term and low-dose administration of Dorzagliatin could slow down the increase of the fasting blood glucose levels in GK rats.

The data in Fig. 2 and Fig. 3 show that, in the Morris water maze spatial memory test, compared with the Wistar rat-solvent group, the GK rat-solvent group showed a significant reduction in both the time spent in the original platform quadrant (Fig. 2; mean + SEM for the Wistar rat-solvent group: 22.17 s ± 1.21 s; mean + SEM for the GK rat-solvent group: 12.16 s ± 2.18 s, **P < 0.01, one-way ANOVA, Dunnett's multiple comparison test) and the swimming distance (Fig. 3; mean + SEM for the Wistar rat-solvent group: 4.45 m ± 0.31 m; mean + SEM for the GK rat-solvent group: 2.25 m ± 0.36 m, **P < 0.01, one-way ANOVA, Dunnett's multiple comparison test).

Compared with the Wistar rat-solvent group, the GK rat-Dorzagliatin group showed no significant difference in the time spent in the original platform quadrant (Fig. 2; mean + SEM for the Wistar rat-solvent group: 22.17 s ± 1.21 s; mean + SEM for the GK rat-Dorzagliatin group: 20.1 s ± 2.56 s, P = 0.7003, one-way ANOVA, Dunnett's multiple comparison test) and the swimming distance (Fig. 3; mean + SEM for the Wistar rat-solvent group: 4.45 m + 0.31 m; mean + SEM for the GK rat-Dorzagliatin group: 4.21 m ± 0.64 m, P = 0.9039, one-way ANOVA, Dunnett's multiple comparison test).

Compared with the GK rat-solvent group, the GK rat-Dorzagliatin group showed a significant increase in both the time spent in the original platform quadrant (Fig. 2; mean + SEM for the GK rat-solvent group: 12.16 s + 2.18 s; mean + SEM for the GK rat-Dorzagliatin group: 20.1 s ± 2.56 s, #P < 0.05, one-way ANOVA, Dunnett's multiple comparison test) and the swimming distance (Fig. 3; mean + SEM for the GK rat-solvent group: 2.25 m ± 0.36 m; mean + SEM for the GK rat-Dorzagliatin group: 4.21 m ± 0.64 m, ##P < 0.01, one-way ANOVA, Dunnett's multiple comparison test).

These data indicate that, the GK rat-solvent group exhibited spatial memory impairment in the Morris water maze spatial memory test at 33 weeks of age, and the long-term treatment with Dorzagliatin had a positive effect on protecting the cognitive function of GK rats.

### Example 2: Dorzagliatin prevented the decrease in insulin receptor (IR) protein expression in the hippocampus of GK rats.

Animal studies have shown that insulin receptors (IRs) are most abundant in the hippocampus, cerebral cortex, striatum, and cerebellum. The widespread distribution of these receptors in the brain indicates that insulin signaling plays a series of important roles, possibly involving the expression of N-methyl-D-aspartate (NMDA) receptors. NMDAreceptors are the most important excitatory glutamate-gated ion channels in the brain, mediating synaptic transmission and plasticity, and regulating various brain functional activities such as neurodevelopment, learning, and memory. Changes in insulin signaling in the central nervous system may accelerate brain aging, regulate neuroplasticity, and participate in the neurodegenerative processes. Low insulin receptor expression levels have been observed in the neuropathology of AD patients. Decreased insulin receptor expression in the hippocampus of rats leads to the downregulation of NMDA receptor protein expression and spatial learning impairment. Therefore, the effect of Dorzagliatin on the hippocampal insulin signaling pathway was evaluated by measuring the protein expression level of insulin receptors in the hippocampus of GK rats.

### Method

GK rats were euthanized after 37 weeks of treatment, i.e., when the rats were 43 weeks old, and hippocampal tissue of the GK rats was collected for western blotting to detect the protein expression levels of the biomarkers.

### (I) Sampling of tissue

Animals under anesthesia were rapidly decapitated and their brains were removed in a hypothermic environment. The hippocampal tissue was separated on ice, placed in cryovials, flash-frozen in liquid nitrogen, and then stored in a -80 °C freezer for later use.

### (II) Protein extraction and quantification

(1) The tissue homogenizer (TissueLyser II high-throughput tissue homogenizer, QIAGEN) was precooled at -20 °C. The hippocampal tissue was taken out from the -80 °C freezer. Protein lysis buffer (RIPA buffer (Solarbio, Cat. R0010), protease inhibitor (bimake, Cat. B14001) and serine protease inhibitor (Solarbio, Cat. P0100) were added at a ratio of 100:1)) was added, and the tissue was lysed at low temperature to give a protein homogenate.
(2) The lysed protein homogenate was centrifuged at 4 °C and 12,000 rpm for 30 minutes using a benchtop high-speed refrigerated centrifuge (Thermo Fisher Scientific, Sorvall ST8R).
(3) The protein supernatant after the centrifugation was collected into a new cryovial, and an appropriate amount of the protein supernatant was taken to determine the concentration using a Pierce^{™} BCA Protein Assay Kit (Thermo Fisher Scientific, Cat. 23227).

### (III) Preparation of denaturated protein samples

Based on the protein concentration determined in step (II), an appropriate amount of protein lysis buffer was added to adjust the protein concentration of each group to a suitable level. An equal amount of twofold concentrated protein loading buffer was added, and the resulting mixture was vortexed to mix well. The proteins were denatured in a 95 °C constant-temperature metal bath for 10 minutes to give a denatured protein sample, which was cooled at low temperature for later use.

### (IV) Gel electrophoresis of proteins

The denatured protein samples prepared in step (III) were subjected to SDS-polyacrylamide gel electrophoresis. After the samples were loaded, the electrophoresis was started at 70V. When the protein molecular weight standard bands became more dispersed, the voltage of the electrophoresis was adjusted to 110V. The electrophoresis was stopped before the bromophenol blue reached the plastic bottom.

### (V) Membrane transfer

The gel obtained after the electrophoresis in step (IV) was used for membrane transfer. Two insulin receptor (IR) isoforms (IR-A protein and IR-B protein) and the internal reference protein GAPDH were transferred to a membrane using a semi-dry transfer method. The membrane was assembled in the following order: negative electrode-filter paper-gel-PVDF membrane-filter paper-positive electrode. The membrane transfer was performed at a constant current of 0.2 A at room temperature for 65 minutes.

After the membrane transfer was completed, the PVDF membranes were blocked with non-fat milk at room temperature for 1 hour. The blocked membranes were washed with Tris-buffered saline containing 0.1% Tween^{®} 20 detergent (TBST), and incubated with a corresponding primary antibody at 4 °C on a low-speed shaker overnight. The primary antibodies were removed, and then the membranes were washed with TBST to remove the unbound primary antibodies. Then, the membranes were incubated with a horseradish peroxidase-labeled goat anti-mouse IgG (H+L) (Yisheng, Cat. 33201ES60)/horseradish peroxidase-labeled goat anti-rabbit IgG (H+L) (Yisheng, Cat. 33101ES60P) secondary antibody at room temperature on a low-speed shaker for 2 hours. The secondary antibodies were removed, and then the membranes were washed with TBST to remove the secondary antibodies that had not bound to the primary antibodies. The eluted PVDF membranes were scanned using an electrochemiluminescence (ECL) method with a contact-based non-destructive quantitative imaging instrument (E-blot) to give a western blot image.

Calculation of the relative expression level of a protein: The western blot images were quantified using an image processing software ImageJ, and the gray value of a target band was measured and used to characterize the protein expression level of the target band.

Target protein normalization: The gray value of a target protein was divided by the gray value of the internal reference protein GAPDH to perform normalization, which characterized the relative expression level of the target band. Statistical analysis was performed on the normalized value of each target band.

### Results

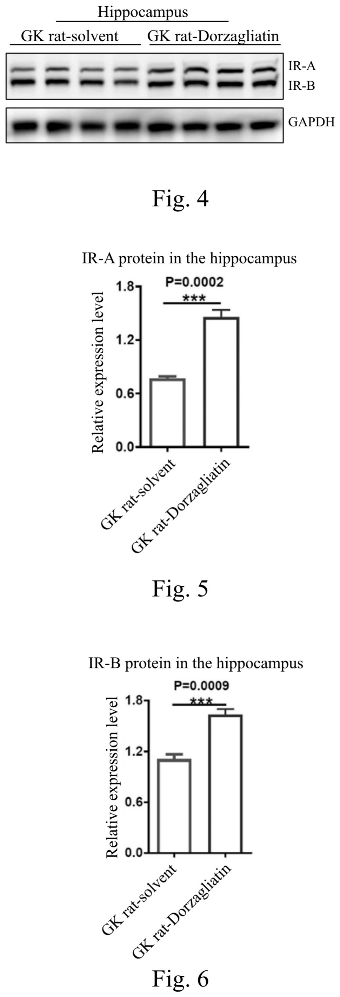

Fig. 4 shows the western blot bands of two insulin receptor isoforms (IR-A protein and IR-B protein) in the hippocampus of the GK rat-solvent group and the GK rat-Dorzagliatin group (4 rats in each group) and the corresponding internal reference protein GAPDH. Fig. 5 and Fig. 6 show the results of statistical analysis of the normalized data of the gray values of IR-A protein and IR-B protein bands in Fig. 4, respectively.

The data in Fig. 5 and Fig. 6 show that the normalized values of IR-A protein and IR-B protein in the hippocampus of the GK rat-Dorzagliatin group were significantly higher than those of the GK rat-solvent group (Fig. 5; mean ± SEM for the GK rat-solvent group: 0.77 + 0.02; mean ± SEM for the GK rat-Dorzagliatin group: 1.46 + 0.08, ***P < 0.001, unpaired Student's t test), (Fig. 6; mean ± SEM for the GK rat-solvent group: 1.11 + 0.06; mean + SEM for the GK rat-Dorzagliatin group: 1.64 ± 0.06, ***P < 0.001, unpaired Student's t test).

These data indicate that the relative expression levels of the two insulin receptor isoforms in the hippocampus of the GK rat-Dorzagliatin group were significantly higher than those of the GK rat-solvent group.

### Example 3: Dorzagliatin stabilized the protein expression levels of the glucose transporters in the hippocampus of GK rats

Neurons are energy-intensive cells, with most of the energy used for generating action potentials, synaptic signaling, and the biosynthesis of neurotransmitters. Glucose is the primary energy source for brain cells, and its transmembrane transport is regulated by the glucose transporter (GLUT) family. GLUT1 and GLUT3 are the two subtypes with the highest affinity for glucose, among which neurons primarily take up glucose via GLUT3, while GLUT1 is the main glucose transporter in astrocytes. GLUT1 can also transport lactate to neurons as an alternative energy source during hypoglycemia. The regulation of neuronal metabolism and energy production are essential for cognition and memory, and depend on the insulin stimulation of GLUTs. Clinical studies have shown that decreased metabolism in certain brain regions of patients with Alzheimer's disease (AD) and type 2 diabetes mellitus (T2DM) may be attributed to reduced expression of GLUT1 and GLUT3 in different brain regions. Reduced expression of GLUT1 and GLUT3 and decreased glucose metabolism in brain have also been observed in rodent models of AD and T2DM. Here, we evaluated the effects of Dorzagliatin on the hippocampal energy metabolism and insulin signaling pathway by analyzing the protein expression levels of insulin receptors, key synaptic proteins, and glucose transporters in the hippocampus of GK rats.

### Method

The tissue sampling, protein extraction and quantification, preparation of the denaturated protein samples, and protein gel electrophoresis were completed successively using the steps (I) to (IV) of the above example 2.

Then, the gel obtained from the electrophoresis was used for membrane transfer. Biomarkers GLUT1, GLUT3, and GAPDH were transferred to a membrane using a semi-dry transfer method. The membrane was assembled in the following order: negative electrode-filter paper-gel-PVDF membrane-filter paper-positive electrode. The membrane transfer was performed at a constant current of 0.2 A at room temperature for 65 minutes.

After the membrane transfer was completed, the PVDF membranes (GLUT1, GLUT3, GAPDH) were blocked with non-fat milk at room temperature for 1 hour.

Calculation of the relative expression level of a protein: The western blot images were quantified using an image processing software ImageJ, and the gray value of a target band was measured and used to characterize the protein expression level of the target band. The gray value of a target protein was divided by the gray value of the internal reference protein GAPDH to perform normalization, which characterized the relative expression level of the target band. Statistical analysis was performed on the normalized value of each target band.

### Results

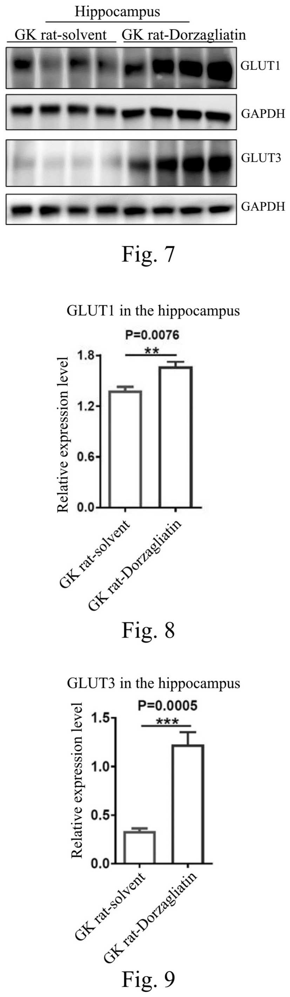

Fig. 7 shows the western blot bands of glucose transporters 1 and 3 (GLUT1 and GLUT3) in the hippocampus of the GK rat-solvent group and the GK rat-Dorzagliatin group (4 rats in each group) and the corresponding internal reference protein GAPDH. Fig. 8 and Fig. 9 show the results of statistical analysis of the normalized data of the gray values of GLUT1 and GLUT3 bands in Fig. 7, respectively.

The data in Fig. 8 and Fig. 9 show that the normalized values of the two glucose transporters in the hippocampus of the GK rat-Dorzagliatin group were significantly higher than those of the GK rat-solvent group (Fig. 8; mean + SEM for the GK rat-solvent group: 1.38 + 0.05; mean + SEM for the GK rat-Dorzagliatin group: 1.67 + 0.06, **P < 0.01, unpaired Student's t test), (Fig. 9; mean + SEM for the GK rat-solvent group: 0.33 + 0.03; mean + SEM for the GK rat-Dorzagliatin group: 1.22 ± 0.13, ***P < 0.001, unpaired Student's t test).

The above data indicate that the relative protein expression levels of the two glucose transporters (GLUT1 and GLUT3) in the hippocampus of the GK rat-Dorzagliatin group were significantly higher than those of the GK rat-solvent group.

### Example 4: Dorzagliatin inhibited the downregulation of two subunits (GluN2A, GluN2B) of the key synaptic protein, the ionotropic glutamate receptor.

### Method

The tissue sampling, protein extraction and quantification, preparation of the denaturated protein samples, and protein gel electrophoresis were completed successively using the steps (I) to (IV) of the above example 2.

Then, the gel obtained from the electrophoresis was used for membrane transfer. Biomarkers GluN2A and GluN2B were transferred to a membrane using a wet transfer method. The membrane was assembled in the following order: negative electrode-sponge-filter paper-gel-PVDF membrane-filter paper-sponge-positive electrode. The membrane transfer was performed at a constant voltage of 100 V in an ice bath for 70 minutes.

After the membrane transfer was completed, the PVDF membranes were blocked with BSA or non-fat milk (GluN2A and GluN2B membranes were blocked with BSA, and GAPDH membrane was blocked with non-fat milk), respectively, at room temperature for 1 hour. The blocked membranes were washed with Tris-buffered saline containing 0.1% Tween^{®} 20 detergent (TBST), and incubated with a corresponding primary antibody at 4 °C on a low-speed shaker overnight. The primary antibodies were removed, and then the membranes were washed with TBST to remove the unbound primary antibodies. Then, the membranes were incubated with a horseradish peroxidase-labeled goat anti-mouse IgG (H+L) (Yisheng, Cat. 33201ES60)/horseradish peroxidase-labeled goat anti-rabbit IgG (H+L) (Yisheng, Cat. 33101ES60P) secondary antibody at room temperature on a low-speed shaker for 2 hours. The secondary antibodies were removed, and then the membranes were washed with TBST to remove the secondary antibodies that had not bound to the primary antibodies. The eluted PVDF membranes were scanned using an electrochemiluminescence (ECL) method with a contact-based non-destructive quantitative imaging instrument (E-blot) to give a western blot image.

Calculation of the relative expression level of a protein: The western blot images were quantified using an image processing software ImageJ, and the gray value of a target band was measured and used to characterize the protein expression level of the target band. Target protein normalization: The gray value of a target protein was divided by the gray value of the internal reference protein GAPDH to perform normalization, which characterized the relative expression level of the target band. Statistical analysis was performed on the normalized value of each target band.

### Results

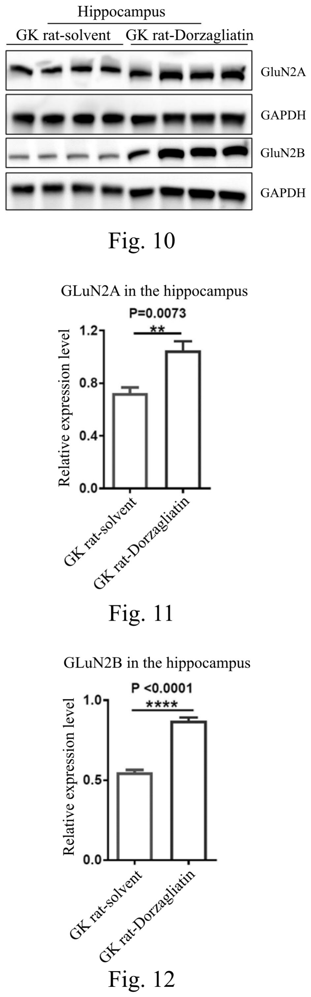

Fig. 10 shows the western blot bands of the subunits (GluN2A and GluN2B) of the ionotropic glutamate receptor in the hippocampus of the GK rat-solvent group and the GK rat-Dorzagliatin group (4 rats in each group) and the corresponding internal reference protein GAPDH. Fig. 11 and Fig. 12 show the results of statistical analysis of the normalized data of the gray values of GluN2A and GluN2B bands in Fig. 10, respectively.

Fig. 11 and Fig. 12 show that the normalized values of the two subunits of the ionotropic glutamate receptor in the hippocampus of the GK rat-Dorzagliatin group were significantly higher than those of the GK rat-solvent group (Fig. 11; mean + SEM for the GK rat-solvent group: 0.72 + 0.04; mean + SEM for the GK rat-Dorzagliatin group: 1.05 + 0.07, **P < 0.01, unpaired Student's t test), (Fig. 12; mean + SEM for the GK rat-solvent group: 0.55 + 0.02; mean + SEM for the GK rat-Dorzagliatin group: 0.87 ± 0.02, ****P < 0.0001, unpaired Student's t test), indicating that the relative protein expression levels of the two subunits (GluN2A and GluN2B) of the ionotropic glutamate receptor in the hippocampus of the GK rat-Dorzagliatin group were significantly higher than those of the GK rat-solvent group.

In summary, in the above examples, long-term treatment of Dorzagliatin in the spontaneously occurring type II diabetes rat model GK rats can prevent the elevated fasting blood glucose and memory decline in GK rats. GK rats treated in the solvent group showed significantly shorter swimming distances and time spent in the original platform quadrant in the Morris water maze spatial memory test, indicating that the spatial memory function was impaired compared with the Wistar rats treated in the solvent group. Dorzagliatin increased the swimming distances and time spent in the original platform quadrant in the Morris water maze spatial memory test, indicating that the spatial memory function was protected compared with the GK rats treated in the solvent group. Long-term treatment of Dorzagliatin can prevent the decrease in the protein expression of insulin receptors in the hippocampus of GK rats and stabilize the protein expression level of glucose transporters in the hippocampus of GK rats. Long-term treatment of Dorzagliatin can also inhibit the downregulation of two subunits of the key synaptic protein, the ionotropic glutamate receptor, suggesting that this may be a novel mechanism by which Dorzagliatin protects the memory function.

Therefore, the glucokinase activator Dorzagliatin is a potential drug for preventing, alleviating, or treating cognitive impairment or a neurodegenerative disease (e.g., mild cognitive impairment, Alzheimer's disease) in a subject, or delaying the progression of cognitive impairment or a neurodegenerative disease in a subject, or reducing the risk of developing cognitive impairment or a neurodegenerative disease in a subject. A prodrug of Dorzagliatin, which can be converted into Dorzagliatin in the body (e.g., in the gastrointestinal tract and intestinal cells), and then absorbed into the circulatory system, is also a potential drug for preventing, alleviating, or treating cognitive impairment or a neurodegenerative disease (e.g., mild cognitive impairment, Alzheimer's disease) in a subject, or delaying the progression of cognitive impairment or a neurodegenerative disease in a subject, or reducing the risk of developing cognitive impairment or a neurodegenerative disease in a subject.

The above is a further detailed description of the present disclosure in connection with the specific alternative embodiments, and the specific embodiments of the present disclosure are not limited to the description. For those skilled in the art, without departing from the concept of the present disclosure, some simple deductions or substitutions can be made, which should be regarded as falling within the protection scope of the present disclosure.

## Claims

1. Use of a glucokinase activator or a prodrug thereof, or a pharmaceutically acceptable salt, an isotope-labeled form, a crystal form, a hydrate, a solvate, a diastereomer or an enantiomer thereof in the manufacture of a medicament for the prevention, alleviation or treatment of cognitive impairment or a neurodegenerative disease in a subject, or delaying the progression of cognitive impairment or a neurodegenerative disease in a subject, or reducing the risk of developing cognitive impairment or a neurodegenerative disease in a subject.

2. The use according to claim 1, wherein the glucokinase activator is a compound represented by the following formula, or a pharmaceutically acceptable salt, an isotope-labeled form, a crystal form, a hydrate, a solvate, a diastereomer or an enantiomer thereof: 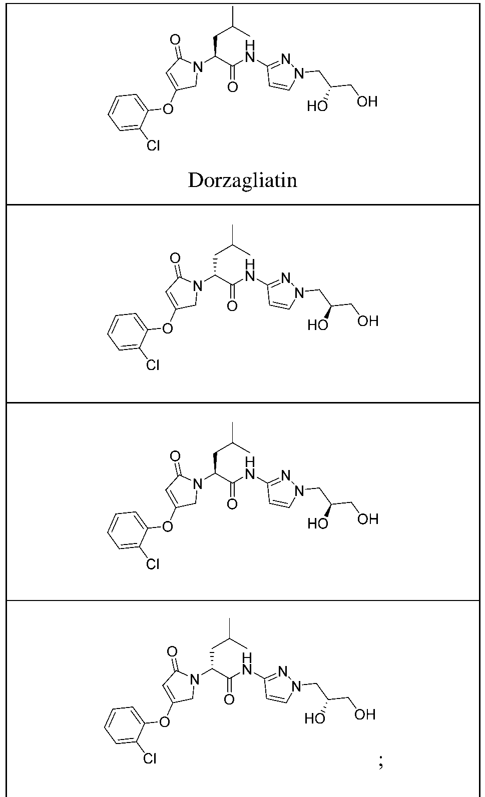 alternatively, the glucokinase activator is Dorzagliatin, or a pharmaceutically acceptable salt thereof.

3. The use according to claim 1, wherein the glucokinase activator is a prodrug of Dorzagliatin, or a pharmaceutically acceptable salt, an isotope-labeled form, a crystal form, a hydrate, a solvate, a diastereomer or an enantiomer thereof;
alternatively, the glucokinase activator is a prodrug of Dorzagliatin, or a pharmaceutically acceptable salt thereof;
yet alternatively, the prodrug of Dorzagliatin is a compound of formula (I), or an isotope-labeled form, an enantiomer, a diastereomer, or a pharmaceutically acceptable salt thereof: 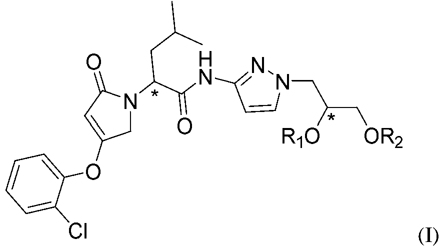 wherein
* indicates a chiral center,
R₁ is selected from H, -C(O)R₆, -C(O)OR₆, -C(O)NR₇R₈, -S(O)ₘR₆, -S(O)ₘOR₆ and -S(O)ₘNR₇R₈;
R₂ is selected from -C(O)R₃, -C(O)OR₃, -C(O)NR₄R₅, -S(O)ₘR₃, -S(O)ₘOR₃ and -S(O)ₘNR₄R₅;
or R₁ and R₂ are connected to form -CHR_{d}-, -SiR_{d}Rₑ-, -C(O)-, -S(O)₁₋₂-, -P(O)OR_{d}-, or -CR_{d}Rₑ-CR_{d}Rₑ₋;
R₃ is independently selected from H, C₁₋₆ alkyl, C₂₋₆ alkenyl, C₂₋₆ alkynyl, C₁₋₆ haloalkyl, C₃₋₇ cycloalkyl, 3-to 7-membered heterocyclyl, C₆₋₁₀ aryl and 5- to 10-membered heteroaryl, and R₃ is optionally substituted with 1, 2, 3, 4 or 5 R groups;
R₄ and R₅ are independently selected from H, C₁₋₆ alkyl, C₂₋₆ alkenyl, C₂₋₆ alkynyl and C₁₋₆ haloalkyl; or, R₄, R₅ and the N atom are taken together to form 3- to 7-membered heterocyclyl;
R₆ is independently selected from H, C₁₋₆ alkyl, C₂₋₆ alkenyl, C₂₋₆ alkynyl and C₁₋₆ haloalkyl;
R₇ and R₈ are independently selected from H, C₁₋₆ alkyl, C₂₋₆ alkenyl, C₂₋₆ alkynyl and C₁₋₆ haloalkyl; or, R₇, R₈ and the N atom are taken together to form 3- to 7-membered heterocyclyl;
R is independently selected from H, -L-halogen, -L-CN, -L-NO₂, -L-ORₐ, -L-SRₐ, -L-NR_{b}R_{c}, -L-C(O)ORₐ, - L-C(O)NR_{b}R_{c}, -L-S(O)ₘORₐ, -L-S(O)ₘNR_{b}R_{c}, C₁₋₆ alkyl, C₁₋₆ haloalkyl, C₂₋₆ alkenyl, C₂₋₆ alkynyl, C₁₋₆ haloalkyl, C₃₋₇ cycloalkyl, 3- to 7-membered heterocyclyl, C₆₋₁₀ aryl, 5- to 10-membered heteroaryl and a side chain of a natural amino acid;
wherein m = 1 or 2;
Rₐ is independently selected from H, C₁₋₆ alkyl, C₂₋₆ alkenyl, C₂₋₆ alkynyl, C₁₋₆ haloalkyl, C₃₋₇ cycloalkyl, 3-to 7-membered heterocyclyl, C₆₋₁₀ aryl and 5- to 10-membered heteroaryl;
R_{b} and R_{c} are independently selected from H, C₁₋₆ alkyl, C₂₋₆ alkenyl, C₂₋₆ alkynyl, C₁₋₆ haloalkyl, C₃₋₇ cycloalkyl, 3- to 7-membered heterocyclyl, C₆₋₁₀ aryl and 5- to 10-membered heteroaryl; or, R_{b}, R_{c} and the N atom are taken together to form 3- to 7-membered heterocyclyl;
R_{d} and Rₑ are independently selected from H, C₁₋₆ alkyl, C₂₋₆ alkenyl, C₂₋₆ alkynyl and C₁₋₆ haloalkyl, or, R_{d}, Rₑ and the C atom are taken together to form =O, =S, C₃₋₇ cycloalkyl or 3- to 7-membered heterocyclyl;
L is selected from chemical bond, -C₁₋₆ alkylene-, -C₂₋₆ alkenylene- and -C₂₋₆ alkynylene-.

4. The use according to claim 3, wherein the prodrug of Dorzagliatin or a pharmaceutically acceptable salt thereof is selected from the compounds of the following structures:

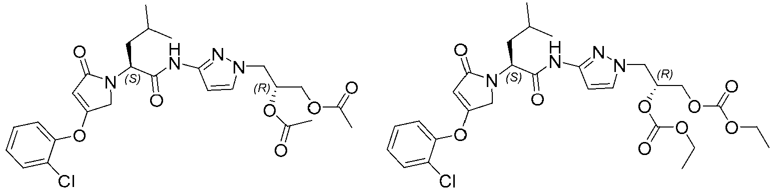

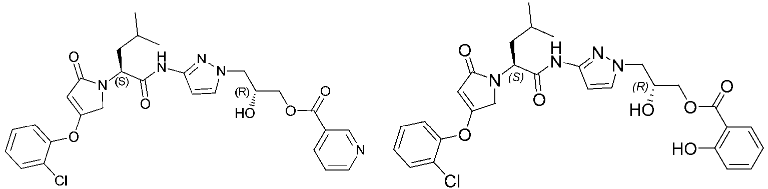

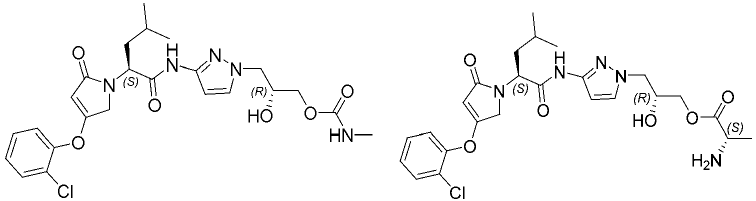

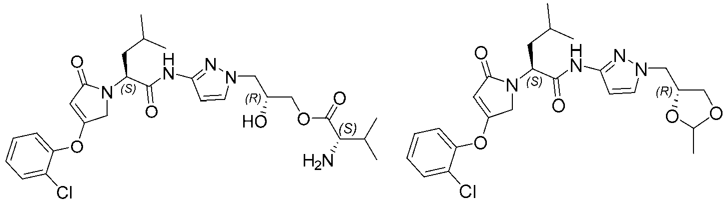

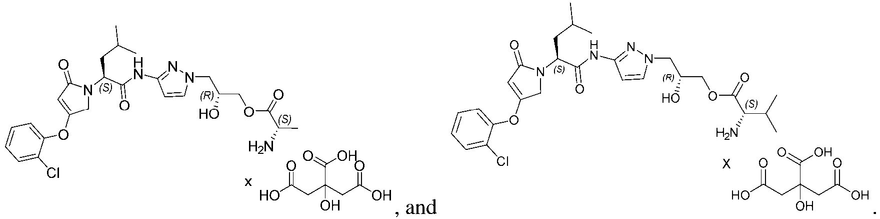

5. The use according to claim 3, wherein the prodrug of Dorzagliatin is a compound of the following structure:

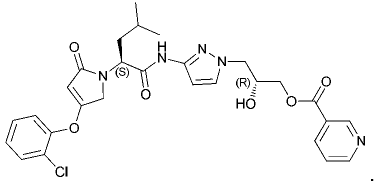

6. The use according to any one of claims 1 to 5, wherein symptoms of the cognitive impairment are decreased attention, decreased language ability, decreased spatial and temporal abilities, decreased reasoning ability, or decreased judgment.

7. The use according to any one of claims 1 to 6, wherein the use is for the prevention, alleviation or treatment of a cognitive impairment-related disease, or delaying the progression of a cognitive impairment-related disease, or reducing the risk of developing a cognitive impairment-related disease.

8. The use according to claim 7, wherein the cognitive impairment-related disease is selected from mild cognitive impairment (MCI), preclinical Alzheimer's disease, Alzheimer's disease (AD), dementia of Alzheimer type, presenile dementia, early-onset Alzheimer's disease, prodromal AD, senile dementia, dementia with Lewy bodies (DLB), dementia of microinfarct, AIDS-related dementia, HIV-associated dementia, Lewy body-related dementia, Down syndrome-related dementia, frontotemporal dementia, Pick's disease, recent and short-term memory impairment, age-related cognitive impairment, age-related memory impairment, drug-related cognitive impairment, immunodeficiency syndrome-related cognitive impairment, vascular disease-related cognitive impairment, schizophrenia-related cognitive impairment, Parkinson's disease-related cognitive impairment, epilepsy-related cognitive impairment, depression-related cognitive impairment, bipolar disorder-related cognitive impairment, obsessive-compulsive disorder-related cognitive impairment, post-traumatic stress disorder, attention deficit disorder, attention-deficit hyperactivity disorder, and learning disabilities;
alternatively, wherein the cognitive impairment-related disease is selected from mild cognitive impairment (MCI), preclinical Alzheimer's disease, Alzheimer's disease, dementia of Alzheimer type, presenile dementia, early-onset Alzheimer's disease, prodromal AD, senile dementia, dementia with Lewy bodies, dementia of microinfarct, AIDS-related dementia, HIV-associated dementia, Lewy body-related dementia, Down syndrome-related dementia, and vascular disease-related cognitive impairment;
alternatively, the cognitive impairment-related disease is mild cognitive impairment or Alzheimer's disease.

9. The use according to any one of claims 1 to 8, wherein the neurodegenerative disease is selected from at least one of the following diseases: Parkinson's disease, Alzheimer's disease, Prion diseases, motor neuron disease (MND) such as amyotrophic lateral sclerosis (ALS), Huntington's disease (HD), spinocerebellar ataxia (SCA), spinal muscular atrophy (SMA), Friedreich's ataxia, Lewy body disease, epilepsy, encephalitis, hydrocephalus, stroke, chronic traumatic encephalopathy (CTE); synucleinopathies; tauopathies; spongiform encephalopathy; familial amyloid polyneuropathy; hereditary cerebral hemorrhage with amyloidosis-Dutch type; cerebral amyloid angiopathy; corticobasal degeneration; Pick's disease; progressive supranuclear palsy; Creutzfeldt-Jakob disease; Gerstmann syndrome; fatal familial insomnia; kuru; bovine spongiform encephalopathy; scrapie; chronic wasting disease; the Lewy body variant of Alzheimer's disease; diffuse Lewy body disease; dementia with Lewy bodies; multiple system atrophy; neurodegeneration with brain iron accumulation, type I; diffuse Lewy body disease; frontotemporal lobar degeneration; hereditary dentatorubral-pallidoluysian atrophy; Kennedy's disease; Alexander disease; Cockayne syndrome; and hereditary cerebral amyloid angiopathy, Icelandic type;
alternatively, the neurodegenerative disease is selected from Parkinson's disease, Alzheimer's disease, Huntington's disease (HD), and motor neuron disease (MND) such as amyotrophic lateral sclerosis (ALS);
alternatively, the neurodegenerative disease is Alzheimer's disease.

10. The use according to any one of claims 1 to 9, wherein the subject has one or more indications selected from: type I diabetes, type II diabetes, maturity-onset diabetes of the young (MODY), gestational diabetes, impaired glucose tolerance, abnormal fasting blood glucose level, hyperglycemia, postprandial hyperglycemia, overweight, obesity, hypertension, cardiovascular disease, insulin resistance and/or metabolic syndrome;
alternatively, the subject has one or more indications selected from: type I diabetes, type II diabetes, maturity-onset diabetes of the young (MODY), gestational diabetes, impaired glucose tolerance, abnormal fasting blood glucose level, hyperglycemia, postprandial hyperglycemia, insulin resistance and/or metabolic syndrome;
alternatively, the subject has one or more indications selected from: type I diabetes, type II diabetes and insulin resistance.

11. Use of a pharmaceutical composition in the manufacture of a medicament for the prevention, alleviation or treatment of cognitive impairment or a neurodegenerative disease in a subject, or delaying the progression of cognitive impairment or a neurodegenerative disease in a subject, or reducing the risk of developing cognitive impairment or a neurodegenerative disease in a subject, wherein the pharmaceutical composition comprises the glucokinase activator according to any one of claims 1 to 10 or a prodrug thereof, or a pharmaceutically acceptable salt, an isotope-labeled form, a crystal form, a hydrate, a solvate, a diastereomer or an enantiomer thereof, and optionally one or more pharmaceutically acceptable excipients.
